# EUROPEAN PATENT SPECIFICATION

(11) **EP 2 347 421 B1**
(45) Date of publication and mention of the grant of the patent: **04.09.2013**
(21) Application number: 09792948.3
(22) Date of filing: 24.09.2009
(51) Int. Cl.: G21F 5/018, A61M 5/145, A61M 5/32

(54) **DROP-IN PIG INJECTOR**
DROP-IN-PIG-INJEKTOR
INJECTEUR A RACLEUR DE CHUTE DE DOSE

(30) Priority: 30.09.2008 US 194749 P
(43) Date of publication of application: 27.07.2011
(60) Divisional application: 12197802.7
(73) Proprietor: Mallinckrodt LLC, Hazelwood, MO 63042 (US)
(72) Inventor: WAGNER, Gary, S., Independence Kentucky 41051 (US)
(74) Representative: Chettle, Adrian John
(86) International application number: PCT/US2009/058210
(87) International publication number: WO 2010/039573

(56) References cited:
- US-A- 6 159 144
- US-A1- 2007 129 591

## Description

### FIELD OF THE INVENTION

The invention relates generally to handling radioactive substances and, more particularly, to transporting radioactive substances and/or injecting radioactive substances into a patient.

### BACKGROUND

The transportation and administration of radioactive substances (e.g., radiopharmaceuticals) may often present issues regarding shielding clinicians and surrounding environments from radiation. Accordingly, radiopharmaceuticals and other radioactive substances are typically transported and stored in containers that prevent or reduce the likelihood of a significant amount of radiation reaching the surrounding environment. For example, radiopharmaceuticals may be transported in a radiation-shielded containment, colloquially referred to as a "pig."

In accordance with typical shielding concerns, the life cycle of radiopharmaceuticals generally includes draw-up of the radiopharmaceutical or other radioactive fluid into a syringe, calibration, and placement of the syringe into a radiation-shielded containment (e.g., a pig) at a supplier facility, transporting the syringe in the radiation-shielded containment to a treatment facility, removal of the syringe from the radiation-shielded containment, injection of the radiopharmaceutical into a subject, returning the syringe to the radiation-shielded containment, and return of the radiation-shielded containment and syringe to the supplier facility. Typically, the radiation-shielded containment includes radiation shielding that completely surrounds the radiopharmaceutical to prevent or reduce the likelihood of radiation escaping into the surrounding environment. However, a potential of exposing a clinician and/or the surrounding environment to radiation may still exist. For example, the radiation-shielded containment may be opened to access the syringe, to identify the syringe, or to inspect the condition of the syringe. Accordingly, the surrounding environment may be exposed to radiation when the radiation-shielded containment is opened. Further, such a shielded containment may create additional steps in administering the radiopharmaceutical. For example, during administration of the radiopharmaceutical, the clinician may remove the syringe from the radiation-shielded containment, inject a patient, and return the syringe to the containment. Accordingly, the use of a shielded containment may add to the complexity of administering a radiopharmaceutical (e.g., removing and returning the syringe).
US-A-6159144 discloses a containment for a syringe adapted to prevent unwanted emissions of radiation.

### SUMMARY

According to the invention there is provided a fluid delivery system, comprising: a containment comprising a body, wherein said body comprises first and second chambers and a passage that extends between said first and second chambers; a first syringe disposed within said first chamber; and medical tubing comprising first and second conduit sections, wherein said first conduit section is interconnected with said first syringe, wherein said medical tubing extends through said passage, and wherein said second conduit section is disposed within said second chamber and is fluidly interconnected with said first conduit section, said medical tubing being adapted for extraction out of said containment for a fluid delivery operation whilst remaining connected to said syringe.

A number of feature refinements and additional features are separately applicable to the present invention. These feature refinements and additional features may be used individually or in any combination. The first syringe may be loaded with an appropriate fluid and the medical tubing may be mounted to a discharge nozzle of the first syringe. With the first syringe and the medical tubing being positioned within an opened containment, the containment may be closed and/or sealed for transport to an appropriate end-use location.

The medical tubing may be of any appropriate size, shape, configuration, and/or type. The medical tubing may contain a first fluid, and the first syringe may contain a second fluid. This configuration may exist when the containment is in a closed or sealed configuration, for instance where both the first syringe and medical tubing are housed within or enclosed by the containment (e.g., during storage and/or transportation of the fluid delivery system to an end-use location). Each of the first and second fluids may be of any appropriate type. In one embodiment, the first fluid is saline, and the second fluid is a radioactive fluid (e.g., a radiopharmaceutical). A valve may be disposed within the medical tubing and/or in a discharge nozzle of the first syringe to maintain a separated state between the first and second fluids until operation of the first syringe is initiated for a fluid discharge operation (e.g., until a syringe plunger is sufficiently advanced). This valve may be of any appropriate size, shape, configuration, and/or type (e.g., a check valve). Multiple valves may be utilized as well and disposed in any appropriate arrangement.

The containment may be in the form of a radiological containment, where a radioactive material of any appropriate type (e.g., a radiopharmaceutical) is contained in a least one of the first syringe and the medical tubing. First shielding may be provided for or associated with the first chamber. In any case, the first shielding may be of any appropriate size, shape, configuration, and/or type, for instance in the form of radiological shielding. By way of example, the first shielding may include lead, tungsten, tungsten-impregnated plastic, depleted uranium, and/or any other suitable radiation shielding material. The first shielding may be incorporated by the containment in any appropriate manner (e.g., by being formed into the body of the containment; by being separately installed on the body of the containment in any appropriate manner, including by being in the form of one or more removable inserts). One or more shielding layers may be utilized for the first shielding. Multiple shielding layers may be disposed in any appropriate arrangement.

The first shielding may be characterized as extending a full 360° about the first chamber (e.g., surrounding the first chamber in at least one dimension; being of an annular configuration). For instance, the first shielding may extend completely about a longitudinal axis of the containment, which may define a length dimension for the containment. The length dimension of the first chamber may extend along this longitudinal axis as well (e.g., the first shielding may extend about the entirety of a circumference of the first chamber).

The containment may include a cap. This cap may be mounted to the body of the containment in any appropriate manner. In one embodiment, the cap is detachably interconnected with the containment body, such that the cap may be installed on and removed from the containment body (e.g., via a threaded interconnection; using a snap-lock interconnection) repeatedly. In one embodiment, the containment body includes a necked end portion at an end of a second chamber that is disposed within the body of the containment. The cap may be characterized as closing or sealing an interior of the body of the containment (e.g., the second chamber).

The medical tubing may be mounted or attached to (e.g., coupled with) the cap. In one embodiment, an end of a second conduit section is detachably engaged or removably coupled with the cap. Removing the cap from the body of the containment may be used to withdraw at least part of the medical tubing from the interior of the containment, for instance for an injection (the medical tubing remaining fluidly interconnected with the first syringe during this withdrawal). The medical tubing may interact with the cap in any appropriate manner. In one embodiment, the cap includes a protrusion, and an open end of the second conduit section (including where an end of the second conduit section includes a fitting or connector of any appropriate type) is disposed over this protrusion to detachably couple the medical tubing and the cap. After the cap has been removed from the containment body, the medical tubing may be disengaged from the cap, for instance, for an injection (e.g., such that the medical tubing may be coupled with a tubing set or the like).

The present invention is characterized by a fluid delivery system that utilizes a containment.

The containment includes a body having first and second ends that are spaced along a longitudinal axis. First and second chambers are disposed within the body, and are spaced along this longitudinal axis. First shielding is provided for this first chamber.

A passage is disposed within the body and extends between the first and second chambers. At least part of the passage proceeds other than collinear with the longitudinal axis and also other than parallel with the longitudinal axis.

The containment may include a body having an access aperture, and a shutter movable relative to the body between first and second shutter positions, where the shutter blocks the access aperture in the first shutter position, and where the shutter exposes the access aperture in the second shutter position (e.g., exposes the access aperture to an interior of the containment).

The first chamber may be aligned with the access aperture.

The access aperture defined in the body of the containment may be adapted to allow a ram of an injection device (e.g., a power injector) to extend through the access aperture to interact with (e.g., interface with and/or mechanically engage) the syringe plunger to move the same in at least one direction (e.g., axially) and for any appropriate purpose (e.g., to discharge fluid from the first syringe).

Any appropriate motion or combination of motions for the shutter may be utilized as the shutter moves between its first shutter position (where it blocks the access aperture) and its second shutter position (where the access aperture is exposed), including an axial or sliding motion (e.g., a movement within a single plane), a pivotal motion (e.g., a movement at least generally about an axis), or the like. The shutter may be biased to its first shutter position in any appropriate manner, for instancing using one or more biasing members of any appropriate size, shape, configuration, and/or type. Multiple biasing members may be disposed in any appropriate arrangement. The shutter may be characterized as being misaligned with the access aperture when in its second shutter position.

A label may at least initially block the access aperture of the containment, and may be of any appropriate size, shape, configuration, and/or type. The access-aperture label may be mounted to an exterior of the containment in any appropriate manner (e.g., adhesively) so as to extend over the access aperture, the shutter may be disposed within an interior of the containment, or both. The access aperture label may include radiological shielding of any appropriate type (e.g., lead, tungsten, tungsten-impregnated plastic, depleted uranium, and/or any other suitable radiation shielding material). Radiological shielding may be incorporated in any appropriate manner in relation to the access aperture label. Some embodiments may not have an access aperture label that includes radiological shielding.

Another function that may be provided by the access aperture label is as a use indicator. A first syringe may be disposed within the first chamber and may include a movable plunger to discharge contents from the first syringe. The access aperture label may be ruptured or punctured by a ram of an injection device (e.g., a power injector) that may interact with the plunger of the first syringe to advance the same (e.g., for a fluid discharge operation). This will provide a visual indication that a ram (or other object) has penetrated the label in an attempt to gain access to an interior of the containment.

Another function that may be provided by the access aperture label is storing information. Any appropriate information may be stored on the access aperture label. Examples of information that may be stored on the access aperture label include without limitation information regarding the contents of the containment (e.g., a radiopharmaceutical), patient identification information, radiopharmaceutical calibration information, warnings, instructions for use, a serial number, identification number, or any combination thereof. Information may be stored in any appropriate manner on the access aperture label (e.g., the access aperture label being in the form of a bar code, an RFID tag, or human readable label). In one embodiment, the access aperture label is machine-readable such that an appropriate device (e.g., bar code scanner, an RFID reader or reader antenna) may be used to retrieve information stored on the access aperture label.

### BRIEF DESCRIPTION OF THE FIGURES

Figure 1 is a block diagram illustrating one embodiment of a radiopharmaceutical lifecycle system that utilizes a radiological containment or pig.

Figure 2 is a perspective view of one embodiment of a radiological containment or pig in a closed position or configuration, and that may be used in the radiopharmaceutical lifecycle system of Figure 1.

Figure 3 is a perspective view of the pig of Figure 2 in an opened position or configuration.

Figure 4 is a plan view of the interior of a second body section of the pig of Figures 2 and 3.

Figure 5 is a plan view of the interior of a first body section of the pig of Figures 2 and 3.

Figure 6 is a plan view of the second body section of Figure 4 in a configuration for providing an injection.

Figure 7 is a schematic of a shutter used by the pig of Figures 2-6.

Figure 8 is a schematic of an alternate embodiment of a shutter that may be used by the pig of Figures 2-6.

Figure 9 is a perspective view of the pig of Figures 2-6 when incorporating a storage compartment.

Figure 10 is a plan view of the interior of an alternate embodiment of a pig that accommodates multiple syringes.

Figure 11 is a block diagram of one embodiment of an imaging system.

Figure 12 is a perspective view of one embodiment of a power injector that may be used by the imaging system of Figure 11.

Figure 13 is a flowchart of one embodiment of a nuclear medicine protocol.

Figure 14 is a flowchart of one embodiment of an injection protocol.

Figure 15 is a block diagram of one embodiment of a fluid delivery system having control logic that may be used to deliver a radioactive fluid.

Figure 16 is one embodiment of an injection protocol that may be used by the fluid delivery system of Figure 15.

Figure 17 is one embodiment of an injection volume determination protocol that may be used by the fluid delivery system of Figure 15.

### DETAILED DESCRIPTION

Various radiological containments (e.g., pigs) are addressed below, along with one or more implementations of such radiological containments. Generally, a "radiological containment" is a structure in which one or more containers (e.g., syringes or vials) may be disposed, where at least one of these containers includes a radioactive material (e.g., a radiopharmaceutical). These radiological containments are oftentimes referred to as "pigs." In certain embodiments addressed herein, a pig includes two body portions or sections that are split along the length of the pig. Specifically, this particular pig may be characterized as utilizing a clamshell configuration that enables a syringe or other medical containers/devices to be stored in one or more internal cavities of the pig, and accessed via separating the body sections. For example, embodiments include a pivot mechanism (e.g., a hinge) that may run the length of the pig, such that the pig may be folded open along its length. As such, first and second body sections of this pig may be characterized as being pivotally coupled to one another. Certain embodiments addressed herein include multiple chambers internal to the pig that enable tubing and/or other devices to be stored in the pig, along with a syringe or other medical container. For example, embodiments may include a first cavity or chamber that houses a syringe (e.g., loaded with a radiopharmaceutical or other radioactive fluid) and a second cavity or chamber that houses tubing that is coupled to the syringe and that may be retracted out of the pig for use in an injection. Certain embodiments enable injecting a subject (e.g., a patient) via the tubing without opening the pig to remove the syringe therefrom. Some embodiments include an aperture that enables access into an internal volume of the pig, and a shutter that prevents or reduces the likelihood of radiation escaping via this aperture. For example and in certain embodiments, the aperture enables a ram (e.g., a ram of a power injector or other injection device) to displace the shutter and to extend into the pig to engage or otherwise interact with the syringe (e.g., a plunger thereof) to discharge fluid therefrom (e.g., by advancing its plunger). Some embodiments include an aperture-sealing label that is disposed over the aperture. For example, the aperture-sealing label may be disposed over the aperture such that puncturing the label provides a visual indication that a power injector ram or the like has at least attempted to interact with the syringe. In some embodiments, the aperture-sealing label includes information encoded on or into the label (e.g., in the form of a radio frequency identification (RFID) tag and/or a bar code label). In some embodiments, the aperture-sealing label incorporates radiation shielding to prevent or reduce the likelihood of radiation escaping the pig via the aperture. Further, certain embodiments addressed herein include a compartment integral to the pig and which may be isolated from interior sections of the pig that house a syringe, tubing, or the like. In some embodiments, the compartment is capable of storing medical devices and equipment. For example, some embodiments may include a compartment integral with the body of the pig that is capable of storing a disposable patient connection set (e.g., IV set). The following includes a discussion of these and other embodiments.

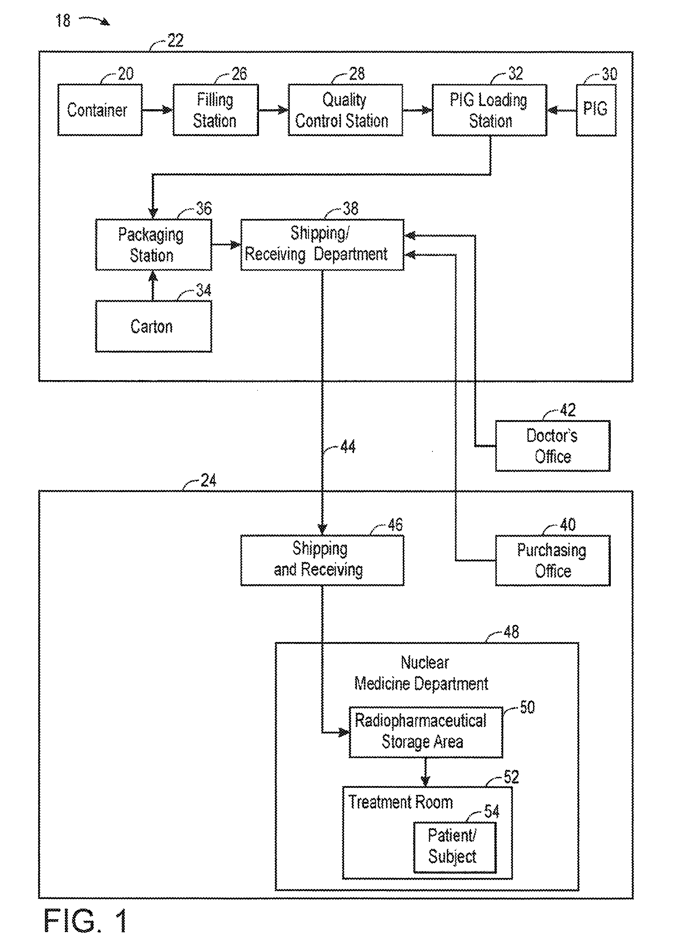

One embodiment of a life cycle of a radiopharmaceutical container and associated pig is shown in Figure 1 as a radiopharmaceutical lifecycle system 18. Referring to Figure 1, containers 20 (e.g., bottles or syringes) may be loaded or filled and packaged at a supplier facility 22 (e.g., a radiopharmacy) that may or may not be remote from a facility 24 (e.g., an end-use facility) in which the radiopharmaceutical is to be used. Within the supplier facility 22, the container 20 may be filled with a radiopharmaceutical at a filling station 26, a quality control check of the radiopharmaceutical may be performed at quality control station 28 and, thereafter, the container 20 may be placed in a radiological containment or pig 30 at a pig loading station 32. The loaded pig 30 may then be packaged either singularly or as a batch in an appropriate shipping carton 34 at a packaging station 36, and the shipping cartons 34 may be temporarily queued or stored in a shipping/receiving department 38. Orders for the radiopharmaceutical containers 20 can be received from various sources, including a purchasing office 40 within the facility 24 (e.g., a healthcare facility), or a doctor's office 42 that may be part of, or independent from, the facility 24. Further, the orders may or may not be associated with a particular patient. Based on the orders, the shipping cartons 34 may enter a distribution channel 44 by which they are delivered to the facility 24, for example, a hospital or other healthcare facility. In the example of Figure 1, the facility 24 has a shipping/receiving area 46 for receiving the cartons 34 of pigs 30 containing containers 20 filled with radiopharmaceuticals. Often (but not always), the cartons 34 may be stored in a nuclear medicine department 48 within the facility 24, which generally includes a radiopharmaceutical storage area 50 and/or treatment room 52 (e.g., diagnostics and/or imaging room). A container 20 may be calibrated and the radiopharmaceutical drawn-up from the container 20 into a syringe in preparation for injection into a patient 54. The correct unit dose volume of radiopharmaceutical to be drawn-up into the syringe generally requires knowing the projected radioactivity level of the radiopharmaceutical at the time the treatment is to be given. To make that determination, it is generally beneficial that one know information such as the radioactivity level at the time the syringe was filled, the filling time and date, the projected treatment time and date, and the rate of decay of the radioactivity of the radiopharmaceutical. Using the projected radioactivity level at the time of treatment and the prescription dosage of the radiopharmaceutical, the correct unit dosage volume can then be determined, and the patient 54 may be injected with the correct dosage of the radiopharmaceutical. As appreciated, treatment may include radiation treatment, diagnostics, and/or imaging.

During the process described above, the radiopharmaceutical is generally enclosed in the pig 30 to prevent or reduce the likelihood of radiation escaping into the surrounding environment. In addition, the pig 30 may also provide a convenient storage/transporting container that reduces the likelihood of damage to the containers 20 (e.g., bottles or syringes). Unfortunately, during the injection process, clinicians may be required to open the pig 30 and subject themselves to radiation from the radiopharmaceutical. For example, the clinician may open the pig 30 to inspect the radiopharmaceutical container 20 or to remove the radiopharmaceutical container 20 from the pig 30 for the injection process. Disclosed below is a radiological containment or pig that may enable transportation and administration of a radiopharmaceutical without the clinician having to open the pig to remove the radioactive material therefrom.

It should be noted that although the following discussion focuses on applications including radiopharmaceuticals, similar embodiments may include other applications including the transportation, storage, and administration of other radioactive substances or fluids. For example, embodiments may include a containment that is used to transport and store nuclear medicines used in the treatment of animals (e.g., livestock). In other words, the embodiments discussed below may be used in any number of nuclear medicine or similar applications that entail the handling and discharge of a radioactive material of any type and in any form.

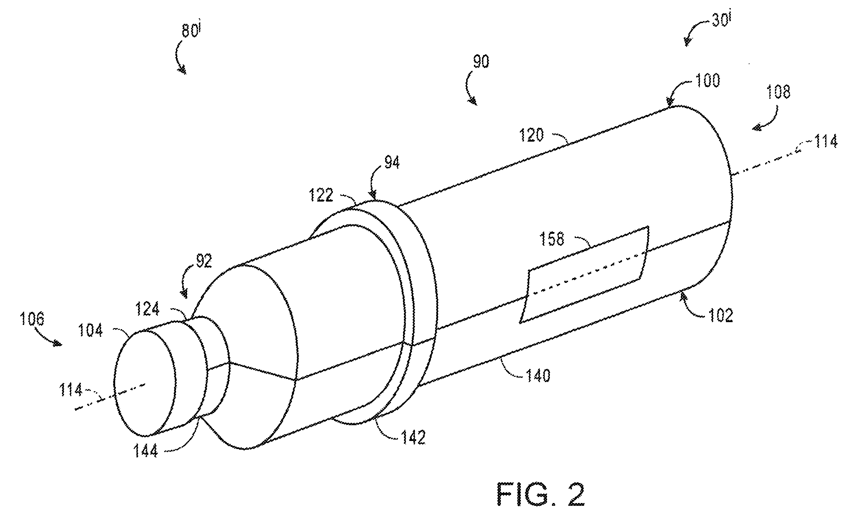

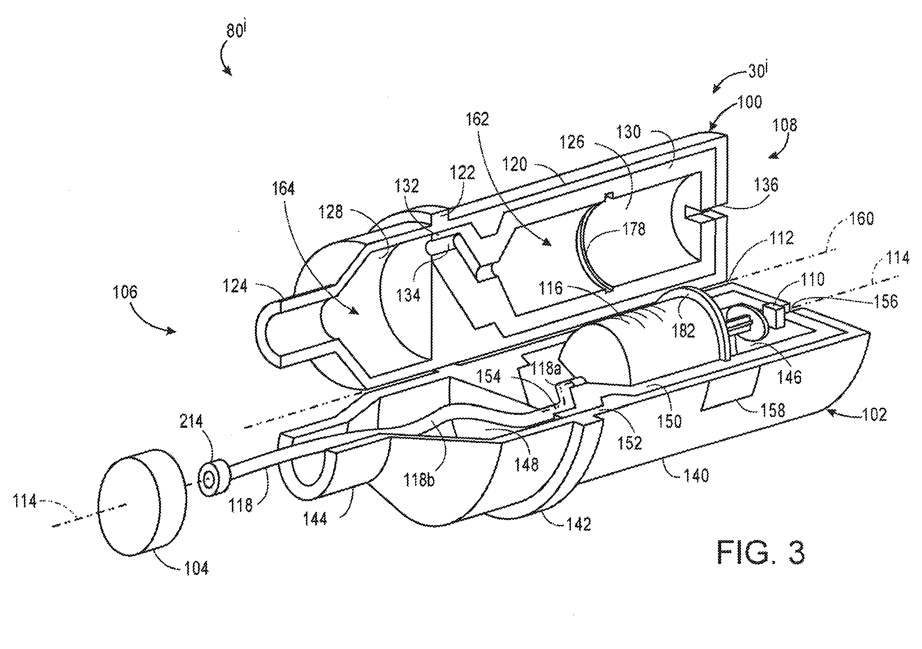

Figures 2 and 3 present perspective views of a particular configuration for a radiological containment or pig 30ⁱ, and which may be used by the radiopharmaceutical lifecycle system 18 of Figure 1 in place of the pig 30. Generally, the pig 30ⁱ houses a syringe 116 and a tube, tubing, or conduit 118 that is fluidly interconnected with the syringe 116. As will be discussed in more detail below, the syringe 116 need not be removed from the pig 30ⁱ in order to deliver a substance from the pig 30ⁱ (e.g., for injection into a patient). In any case, the pig 30ⁱ, syringe 116, and tube 118 may be characterized as a fluid delivery system 80ⁱ.

In the illustrated embodiment, the pig 30ⁱ includes a body 90 having a first end 106 and a second end 108 that are spaced along a longitudinal axis 114. This body 90 includes a first body section 100 and a second body section 102 that are movably interconnected by a pivot mechanism 112 or the like. The pivot mechanism 112 is parallel with the longitudinal axis 114 in the illustrated embodiment. That is, the first body section 100 is movable relative to the second body section 102 at least generally about an axis that is parallel with the longitudinal axis 114 of the pig 30ⁱ. This relative movement provides both open (Figure 3) and closed (Figure 2) configurations for the pig 30ⁱ.

The pig 30ⁱ further includes a cap 104 that may be detachably interconnected with the body 90 at its first end 106. A shutter 110 of the pig 30ⁱ is movably interconnected with the body 90 at its second end 108. The interior of the body 90 may be accessed to at least a certain degree by removing the cap 104 and by moving the shutter 110 relative to the body 90, for instance in preparation of and for providing an injection.

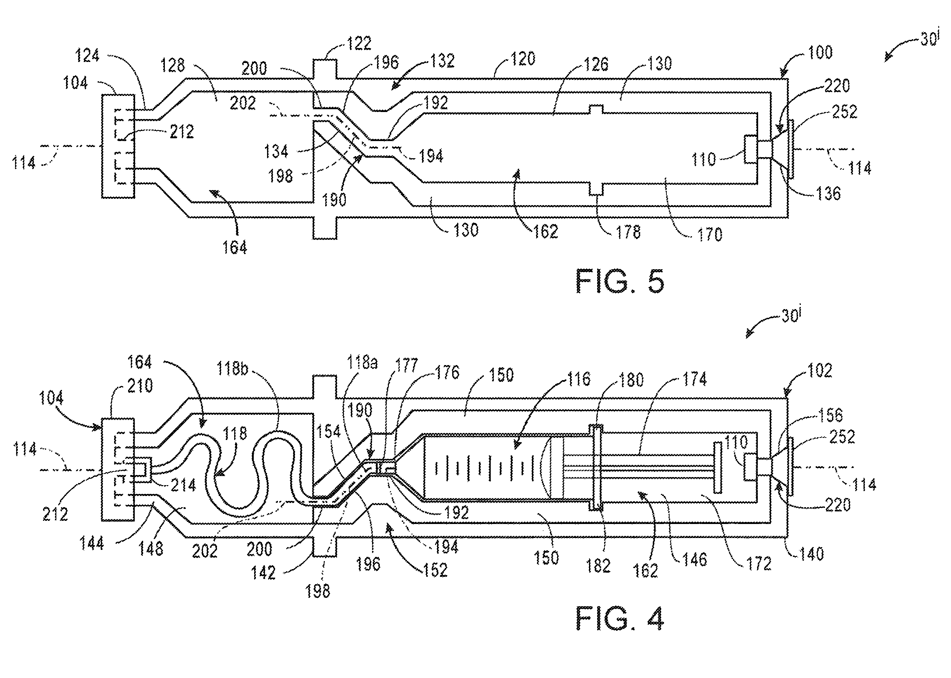

The first body section 100 includes a first body portion 120, a first indexing rib 122, a first neck portion 124, a first cavity portion 126, a second cavity portion 128, a first shielding layer 130, a first cavity separation portion 132, a first internal aperture portion 134, and a first external aperture portion 136. The second body section 102 includes a second body portion 140, a second indexing rib 142, a second neck portion 144, a first cavity portion 146, a second cavity portion 148, a second shielding layer 150, a second cavity separation portion 152, a second internal aperture portion 154, and a second external aperture portion 156. Generally, the first cavity portion 126 of the first body section 100 and the first body portion 146 of the second body section 102 collectively define a first chamber 162 within the pig 30ⁱ (e.g., internally disposed and for receiving the syringe 116). Similarly, the second cavity portion 128 of the first body section 100 and the second cavity portion 148 of the second body section 102 collectively define a second chamber 164 within the pig 30ⁱ (e.g., internally disposed for receiving part of the tube 118 (e.g., Figure 4)). In this regard, a first tube or conduit section 118a of the tube 118 is attached to (e.g., fluidly interconnected with) the syringe 116, which again is in the first chamber 162. The tube 118 extends into the second chamber 164 where a second tube or conduit section 118b of the tube 118 may be stored (e.g., Figure 4) until a fluid delivery operation is to be undertaken in a manner that will be discussed in more detail below.

Figure 2 presents a perspective view of the pig 30ⁱ in a closed position or configuration. For example, the pig 30ⁱ includes the first body section 100 mated with the second body section 102 to form a clamshell or casket-type enclosure. In other words, the first and second body portions 120 and 140 close to enclose or house the syringe 116 and the tube 118. As illustrated, the first body section 100 and second body section 102 are separated along their length or length dimension in a direction that is at least generally parallel to the longitudinal axis 114 of the pig 30ⁱ. For example, the first body section 100 and the second body section 102 may be separated by a horizontal plane that runs parallel and through the longitudinal axis 114 of the pig 30ⁱ. Accordingly, the first body section 100 and the second body section 102 may be substantially symmetrical and may form a top and bottom half, respectively, of the pig 30ⁱ. As illustrated, the first and second body sections 100 and 102 directly oppose one another when the pig 30ⁱ is in the closed position.

In other embodiments, the first body section 100 and the second body section 102 may include alternate geometries. For example, the separation between the first and second body sections 100 and 102 may include a horizontal plane that is parallel to but offset from the longitudinal axis 114 (e.g., above the longitudinal axis 114). Accordingly, the first and second body sections 100 and 102 may not be generally symmetrical; instead, one of these body sections may be larger than the other. Further, embodiments may include separations between the first and second body sections 100 and 102 that include multiple planes running parallel, transverse, or at other angles to the longitudinal axis 114 of the pig 30ⁱ. Moreover, the first and second body sections 100 and 102 may have indentations, extensions, or other features that are mated to one another. For example, the first body section 100 may include a lip that mates with a complementary feature of the second body section 102 to aid in sealing the pig 30ⁱ when it is closed. In any case, what is desirable is for the body 90 of the pig 30ⁱ to be of a configuration such that the body 90 may be moved in at least some respect between open and closed positions to provide open and closed configurations for the pig 30ⁱ. Any appropriate way for selectively maintaining the body 90 in its closed position may be utilized (e.g., cap 104 discussed below; one or more latches; a snap-lock relationship between the first and second body sections 100, 102 when in the closed position).

As further illustrated in the embodiment of Figure 2, the first body section 100 and the second body section 102 collectively define a seam when in the closed position, and a sealing label 158 may be secured to the body 90 and extend over this seam (e.g., a junction or boundary between the first and second body sections 100,102). The sealing label 158 could be used to at least assist in retaining the pig 30ⁱ in its closed position. In any case and in the illustrated embodiment, the sealing label 158 may be in the form of an adhesive label that is secured to the exterior of the pig 30ⁱ. In order to open the pig 30ⁱ, the sealing label 158 may be ruptured, cut, or torn and the pig 30ⁱ subsequently opened. Accordingly, the sealing label 158 may provide a visual indication that the pig 30ⁱ may have been opened, of use of the radiopharmaceutical contained therein, of potential tampering with the pig 30ⁱ, or the like.

Embodiments may include information stored on the above-noted sealing label 158. For example, the sealing label 158 may include patient identification information, radiopharmaceutical calibration information, warnings, instructions for use, a serial number, an identification number, or the like. In this regard, the sealing label 158 may be in the form of a bar code label, an RFID tag, or the like. Information of any type may be stored on the label 158 and in any appropriate manner. Accordingly, a clinician or one or more devices (e.g., an RFID reader) may be used to read the information provided by the label 158. Embodiments may also include one or more of the sealing labels 158. For instance, several labels 158 may be placed at appropriate locations on the pig 30ⁱ to provide additional security, to provide additional/redundant information storage, or the like.

The illustrated embodiment includes a cap 104 disposed on the first and second neck portions 124 and 144 of the pig 30ⁱ when in its closed configuration. The first and second neck portions 124, 144 collectively define a necked end portion 92 for the body 90 when in its closed position, and the cap 104 may be mounted on the necked end portion 92 in any appropriate manner. For instance, the cap 104 may be detachably interconnected with the necked end portion 92 of the body 90 (e.g., via a threaded interconnection, via a snap-lock type interconnection). In any case, the cap 104 closes the first end 106 of the pig 30ⁱ, and may prevent or reduce the likelihood of the first body section 100 and the second body section 102 separating from one another (e.g., opening). Accordingly, the cap 104 may be removed prior to opening the pig 30ⁱ.

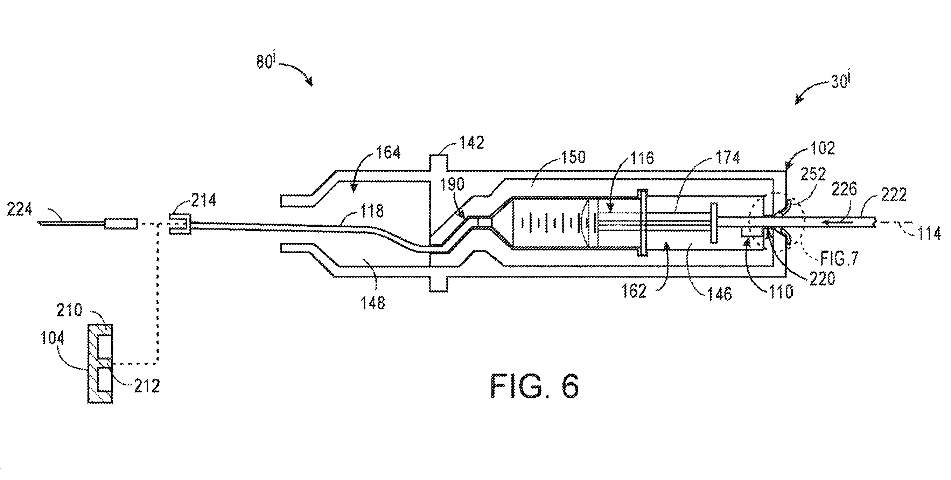

The cap 104 may also include features that enable coupling of the tube 118 to the cap 104. Coupling the tube 118 to the cap 104 may enable simplified access to the tube 118. In one embodiment, the interior surface of the cap 104 includes a projection 212 over which an open end of the second tube section 118b may be disposed (e.g., Figure 6). Removing the cap 104 from the body 90 may then also extract the tube 118 from the second chamber 162, as is discussed in further detail below. Further, an embodiment may include disposing another sealing label 158 over a junction between the cap 104 and the body 90 of the pig 30ⁱ. For example, an additional sealing label 158 may be placed over this junction such that removal of the cap 104 would rupture or tear this particular sealing label 158 to provide a visual indication that the cap 104 may have been removed. Other embodiments may not include the cap 104 coupled to the body 90. For example, one embodiment may include an opening at the first end 106 that is not covered by the cap 104. In other words, the tube 118 may be simply tucked into the pig 30ⁱ without the cap 104 such that the second tube section 118b is exposed to the environment at all times.

The indexing ribs 122 and 142 may provide a reference point when locating (e.g., loading or installing) the pig 30ⁱ relative to one or more other devices. For example and in the illustrated embodiment, the indexing ribs 122 and 142 collectively define a ring-like rib 94 extending about the entire circumference of the body 90 of the pig 30ⁱ. The indexing rib 94 may provide a positional registration function (e.g., to register the position of the pig 30ⁱ relative to another device). Thus, when installing the pig 30ⁱ on an injection device (e.g., a power injector), the indexing rib 94 may be disposed in or engaged with a complementary feature (e.g., annular recess) of the injection device to provide proper alignment of the pig 30ⁱ. In other embodiments, a plurality of the indexing ribs 122 and 142 may be utilized by the pig 30ⁱ. For example, a second set of indexing ribs 122 and 142 may be located on the pig 30ⁱ. Further, the shape and location of the individual ribs 122 and 142 may be varied to accommodate specific applications. Generally, the pig 30ⁱ may include one or more features of any appropriate size, shape, configuration, and/or type to facilitate the use of the pig 30ⁱ with any appropriate device or combination of devices (e.g., to at least reduce the potential of the pig 30ⁱ being incorrectly installed on another device).

Figure 3 illustrates a perspective view of the pig 30ⁱ in an open position. In the open position, the first body section 100 and the second body section 102 are at least partially separated to enable access to regions and components internal to the pig 30ⁱ (e.g., to load, access, and/or remove the syringe 116 and the tube 118). For example and in the illustrated embodiment, the first body section 100 is pivoted (e.g., rotated) relative to the second body section 102 about the pivot mechanism 112. The pivot mechanism 112 pivotally couples the first body section 100 and the second body section 102. Incidentally, "pivotally coupled" or the like, refers to any type of coupling that allows a structure to at least generally undergo a pivoting or pivotal-like motion, including without limitation any coupling that allows a structure or a portion thereof to move at least generally about a certain axis. Representative pivotal couplings that result in a "pivoting" as used herein include the use of a flexing or elastic deformation of a structure or a portion thereof, as well as the use of relative motion between two or more structures that are typically in interfacing relation during at least a portion of the relative movement (e.g., a hinge connection; a ball and socket connection). In the illustrated embodiment, the pivot mechanism 112 includes a pivot axis 160 that is at least generally parallel to the longitudinal axis 114 of the pig 30ⁱ. Accordingly, the pig 30ⁱ includes a clamshell or casket-type configuration that enables the pig 30ⁱ to be opened along its length or length dimension.

The design of the pivot mechanism 112 may be varied to accommodate specific applications. For example and in the illustrated embodiment, the pivot mechanism 112 includes an integral portion of material that extends between the first body section 100 and the second body section 102. Such a pivot mechanism 112 may be referred to as a "living hinge." In other words, the pivot mechanism 112 includes material (e.g., plastic) that couples the first body section 100 and the second body section 102. In such an embodiment, the first body section 100 and the second body section 102 may be molded simultaneously and include a contiguous member (e.g., the pivot mechanism 112) that is formed between the first body section 100 and the second body section 102. In another embodiment, the pivot mechanism 112 may include a separate component that is affixed to the body sections 100 and 102 via adhesive or other fasteners (e.g., a separate hinge or hinges). For instance, the pig 30ⁱ may include one or more pivot mechanisms 112 that are screwed into, glued, or plastic-welded to the first body section 100 and the second body section 102. Notwithstanding the foregoing, any appropriate way of movably interconnecting the first body section 100 and the second body section 102 may be utilized, where the first body section 100 and second body section 102 are movable relative to each other but remain interconnected in at least some respect at all times.

Although the pivot mechanism 112 may provide for movable coupling of the first and second body sections 100 and 102, another embodiment may not include any pivot mechanism 112 at all. For example, an embodiment may include the first body section 100 and the second body section 102 that are completely separated in an opened position and coupled to one another in a closed position. In other words, the first body section 100 and second body section 102 may be completely disassembled from one another when opened (e.g., separately movable relative to each other) and fastened or secured to one another in any appropriate manner (e.g., with a bolt, a ring about the outer surface, or other fastening mechanism(s)) when closed.

Figures 4 and 5 present plan views of the interior of the second body section 102 and the first body section 100, respectively. As previously discussed, the first body section 100 and the second body section 102 may include a plurality of cavity portions 126,128 and cavity portions 146 and 148, respectively. Accordingly, when the pig 30ⁱ is assembled, the first cavity portions 126 and 146 may align to collectively form a first cavity or chamber 162, and the second cavity portions 128 and 148 may align to collectively form a second cavity or chamber 164.

In one embodiment, the first cavity or chamber 162 may be used to house a medical device, such as the syringe 116. For example and as shown in Figure 4, the syringe 116 may be disposed in the first cavity portion 146 of the second body portion 140 of the second body section 102 (also see Figure 3). The pig 30ⁱ may then be closed. Accordingly, the first cavity portions 126 and 146 of the first and second body sections 100,102 may generally include a contour that conforms to the profile of the syringe 116, and that enables the pig 30ⁱ to be closed around the syringe 116. For example and in the illustrated embodiment, the first cavity portions 126 and 146 may include semicircular sections 170 and 172. These semicircular sections 170 and 172 may include a longitudinal portion having a taper at one end. Thus, the semicircular sections 170 and 172 enable the syringe 116 to be disposed at least generally coincident with the longitudinal axis 114. Such an arrangement may then enable a plunger 174 and an outlet 176 of the syringe 116 to be at least generally coincident with the longitudinal axis 114 of the pig 30ⁱ (e.g., the plunger 174 may move along an axial path that coincides with the longitudinal axis 114).

Embodiments of the first cavity portions 126 and 146 may include other features conducive to housing and/or operating the syringe 116. For example and in one embodiment, the first cavity portions 126 and 146 include indentations 178 and 180, respectively, that receive or accept a lip or flange 182 of the syringe 116. In the illustrated embodiment, the indentations 178 and 180 include recessed grooves about the diameter of the semicircular sections 170 and 172, respectively. Accordingly, inserting the flange 182 of the syringe 116 relative to the pig 30ⁱ into the indentations 178 and 180 on the first and second body sections 100, 102 may ensure proper alignment of the syringe 116 and reduce movement of the syringe 116 relative to the pig 30ⁱ when in its closed configuration.

As previously discussed, the pig 30ⁱ may provide shielding of the environment from radiopharmaceuticals or other radioactive substances contained within the pig 30ⁱ. Accordingly and in certain embodiments, the first cavity portions 126 and 146 may include radiation or radiological shielding to prevent or reduce the likelihood of radiation (e.g., radiation generated by a radiopharmaceutical housed in the syringe 116) passing through the pig 30ⁱ, For example and in the illustrated embodiment, the first cavity portions 126 and 146 are lined with or otherwise incorporate a first radiation shielding layer 130 and a second radiation shielding layer 150, respectively. The radiation shielding layers 130 and 150 may include various materials that prevent or reduce the potential of radiation passing through a wall of the pig 30ⁱ. For example and in one embodiment, the radiation shielding layers 130 and 150 may include lead, tungsten, impregnated plastic, or any other suitable shielding material that may enable the pig 30ⁱ to shield the surrounding environment from radiation. In the illustrated embodiment, the shielding layers 130 and 150 collectively shield at least the first chamber 162 of the pig 30ⁱ.

As will be appreciated, the thickness and number of shielding layers 130 and 150 may be varied to enable the pig 30ⁱ to provide appropriate shielding in certain applications. For example and in radiopharmaceutical applications having low levels of radiation, the shielding layers 130 and 150 may include a single layer of material (e.g., lead) disposed proximate to the medical container (e.g., syringe 116) and having an overall thickness of about 0.32 -12.7 mm (0.125 - 0.5 in). However, in other applications where the pig 30ⁱ may be used to transport radiopharmaceuticals or radioactive substances that have an increased level of radiation, the shielding layers 130 and 150 may be increased in thickness and/or increased in number to provide an appropriate amount of shielding. For example, in nuclear medicine applications that include doses of radioactive substances for animals (e.g., livestock), the pig 30ⁱ may include the shielding layers 130 and 150 having a thickness of one-half of an inch or more, or including multiple layers of shielding material/substances.

The arrangement and location of the shielding layers 130 and 150 may be varied to accommodate specific applications. For example and in one embodiment, the shielding layers 130 and 150 may be integral with the first body portion 120 and the second body portion 140. In other words, the shielding layers 130 and 150 may be formed as an innermost layer or region of the cavity portions 126 and 146. For example, the interior may be formed of plastic (e.g., a plastic coating, lining, or insert) over the shielding material. However, in some embodiments, the shielding layers 130 and 150 may be provided as components that are separate from the first body portion 120 and the second body portion 140. In other words, the shielding layers 130 and 150 may include a rigid or semi-rigid inserts that may be appropriately coupled to the first body portion 120 and the second body portion 140. Providing the shielding layers 130 and 150 as inserts may enable the exchange of shielding materials without the need to dispose or exchange the remainder of the pig 30ⁱ (e.g., body portions 120 and 140). Generally, appropriate radiological shielding may be utilized in relation to the first chamber 162 of the pig 30ⁱ and this shielding may be incorporated/implemented in any appropriate manner.

In the illustrated embodiment, the second cavity 164 of the pig 30ⁱ may be configured so as to not utilize any radiation shielding material/substance. However, the entirety or a portion of the second cavity 164 may incorporate a radiation-shielding material. For example, the second cavity portions 128 and/or 148 may be lined with an appropriate radiological shielding material (e.g., lead). Any appropriate radiological shielding may be utilized in relation to the second chamber 164 of the pig 30ⁱ as desired/required, and this radiological shielding may be incorporated/implemented in any appropriate manner.

As previously discussed, embodiments of the pig 30ⁱ may include the second chamber 164. For example, in the illustrated embodiment and when the pig 30ⁱ is closed, the second cavity portion 128 of the first body portion 120 and the second cavity portion 148 of the second body portion 140 may be aligned to collectively define the second chamber 164. In one embodiment, the second cavity portions 128 and 148 may include a semicircular cutout region of the first body portion 120 and the second body portion 140, respectively at the first end 106 of the pig 30ⁱ. Accordingly, the second chamber 164 may be in the form of a hollow chamber (e.g., cylindrical) that is internally disposed and that extends to the first end 106 of the pig 30ⁱ.

In certain embodiments, the second chamber 164 may enable housing and storing medical devices internal to the pig 30ⁱ. For example and in the illustrated embodiment, the second tube section 118b of the tube 118 may be coiled into the second chamber 164 for storage, and extracted subsequently from the second chamber 164 for a fluid delivery operation (e.g., an injection). When extracted, the tube 118 may be coupled to other medical devices (e.g., a patient line connection set) as will be discussed in more detail below with regard to Figure 6. Further, the second chamber 164 may also include other features that are beneficial to operation of the pig 30ⁱ. For example and in the illustrated embodiment, the necked end portion 92 of the pig 30ⁱ may provide for simplified extraction of the tube 118 from within the pig 30ⁱ. For instance, the necked end portion 92 may include a smaller diameter than the second chamber 164, which in turn may reduce the likelihood of the coiled tube 118 being inadvertently withdrawn or extracted from the second chamber 164. Further and as discussed above, the necked end portion 92 may enable the cap 104 to be secured to the pig 30ⁱ to close off the second chamber 164 and, thus, enclose the tube 118 within the pig 30ⁱ.

In certain embodiments, the first chamber 162 and the second chamber 164 of the pig 30ⁱ may be in communication via an aperture or passage that extends through a portion of the first and second bodies 120 and 140. For example and in the illustrated embodiment, the first cavity separation portion 132 includes a first internal aperture portion 134, and the second cavity separation portion 152 includes a second internal aperture portion 154. Accordingly, when the pig 30ⁱ is closed, the aperture portions 134 and 154 collectively define an aperture or passage 190 that extends between the first chamber 162 and the second chamber 164. In the illustrated embodiment, the passage 190 provides a passage for the tube 118 to extend from the outlet 176 of the syringe 116 to the second chamber 164. The passage 190 may include features that are conducive to blocking radiation that is emitted from a medical device (e.g., syringe 116) and/or radiopharmaceuticals disposed in the first chamber 162. For example and in the illustrated embodiment, the passage 190 incorporates an orientation that may prevent or reduce the amount of radiation that passes between the first chamber 162 and the second chamber 164. Specifically, the passage 190 includes multiple bends so as to not provide a straight-line path for radiation to pass between the first chamber 162 and the second chamber 164. In the illustrated embodiment, the passage 190 includes a first passage portion 192 having a first passage axis 194, a second passage portion 196 having a second passage axis 198, and a third passage portion 200 having a third passage axis 202. The arrangement of the axes 194, 198, and 202 are angled different relative to one another at their respective intersections such that there is not a straight-line path between the first and second chambers 162, 164. For example, the first passage axis 194 and the third passage axis 202 may be substantially parallel but offset from each other, and the second passage axis 198 is disposed at an angle to each of the first and third passage axes 194 and 202 (e.g., an included angle of 45 degrees). Further and in the illustrated embodiment, the first passage axis 194 is coincident with the longitudinal axis 114, and the third passage axis 202 is offset from but parallel to the longitudinal axis 114. As such, the second passage axis 198 may be characterized as diverging away from the longitudinal axis 114 progressing from the first passage portion 192 to the third passage portion 200. Accordingly, the passage 190 may be characterized as being of an at least generally Z-shaped configuration that enables the outlet 176 of the syringe 116 to be disposed in or proximate to the first passage portion 192, and that enables the tube 118 to extend from the outlet 176 to the second chamber 164 other than in a straight line. Generally, the passage 190 may be of any appropriate configuration that does not provide a straight-line path from the first chamber 162 to the second chamber 164.

The passage 190 may be lined with radiation shielding to prevent or reduce the potential of the radiation traveling in a straight-line path between the first and second chambers 162 and 164. For example and in the illustrated embodiment, the first shielding layer 130 may extend around the first internal aperture portion 134, and the second shielding layer 150 may extend around the second internal aperture portion 154. Similar to the discussion above regarding the configuration of the shielding layer about the first chamber 162, the shielding layers about the passage 190 may be varied in shape, thickness, composition, and the like to provide an appropriate radiation shield between the first and second chambers 162 and 164. Any appropriate radiological shielding may be utilized in relation to the passage 190, and this shielding may be incorporated/implemented in any appropriate manner.

Turning now to Figure 6, the cap 104 may include various features that enable closing off the second chamber 164 and that enable simplified extraction and retraction of the tube 118. For example and in the illustrated embodiment, the cap 104 includes a lip 210 and a tube engagement feature in the form of a projection 212. The lip 210 includes a ridge about the circumference of the cap 104 that enables the cap 104 to be secured to the pig 30ⁱ. For example, the cap 104 may be detachably secured to the neck portions 124 and 144 of the body portions 100 and 102 to close off the second chamber 164 when the pig 30ⁱ is in its closed configuration. Closing the second chamber 164 may retain the tube 118 in the second chamber 164 (specifically the second tube section 118b) and may protect the tube 118 from the external environment during transportation and storage.

As further illustrated in Figure 6, the tube engagement feature 212 may provide for connection of the tube 118 to the interior of the cap 104, which may simplify extraction of the tube 118 from the second chamber 164. For example and in the illustrated embodiment, the tube engagement feature 212 is in the form of a projection or protrusion that engages a tube connector 214 of the tube 118 (specifically of the second tube section 118b). Thus, the connection between the tube engagement feature 212 and the connector 214 of the tube 118 may enable the tube 118 to be extracted from within the second chamber 164 when the cap 104 is removed and pulled away from the body 90 of the pig 30ⁱ. In other words, the tube 118 may be pulled out automatically via removal of the cap 104 from the body 90, as opposed to a clinician having to reach into the second chamber 164 to extract the tube 118 from the interior of the pig 30ⁱ. Similarly, the tube 118 (e.g., its associated connector 214) may be connected or mounted to the tube engagement feature 212 of the cap 104 prior to replacing the cap 104 on the body 90 of the pig 30ⁱ, and thus may provide for a simplified extraction of the tube 118 at a later time. In certain embodiments, the tube connector 214 may include a quick-connector, or the like, that may couple to a catheter or patient line tubing that is used to deliver the radiopharmaceutical and other substances to a subject. Accordingly, the design of the tube engagement feature 212 on the interior of the cap 104 (e.g., a surface thereof that interfaces with the second chamber 164 when the cap 104 is installed on the body 90), may be varied to accommodate specific types of connectors 214. For example, male and female Luer connectors may be used to connect the cap 104 with the tube 118.

The pig 30ⁱ may include other features that are conducive to the delivery of the radiopharmaceutical or other substances without removing the syringe 116 from the pig 30ⁱ and as addressed above. For example and as illustrated in Figures 4-6, the pig 30ⁱ may include an access or external aperture 220. The external aperture 220 may be in the form of an opening in the body 90 of the pig 30ⁱ that enables a ram 222 of an injection device (e.g., a power injector) to pass into the pig 30ⁱ and engage or otherwise interact with the syringe 116 (e.g., its plunger 174). In the illustrated embodiment, the external aperture 220 includes a first aperture portion 136 on the first body section 100 and a second aperture portion 156 on the second body section 102 that are generally disposed along the longitudinal axis 114 of the pig 30ⁱ. Accordingly, when the pig 30ⁱ is closed, the first and second aperture portions 136 and 156 align to collectively form the external aperture 220.

Passing the ram 222 of the injection device though the external aperture 220 of the pig 30ⁱ and interacting with (e.g., engaging) the plunger 174 of the syringe 116 may force the radiopharmaceutical, or other substances, from the syringe 116 (via the outlet 176) and into/through the tube 118. For example and as illustrated in Figure 6, the tube 118 may be connected to a patient line connection set 224 via the extracted tube 118. Thus, advancing the ram 222 in the direction of arrow 226 in Figure 6 may eject the radiopharmaceutical or other substance out of the syringe 116 and into the patent line connection set 224 via the tube 118. In the illustrated embodiment, the aperture 220 includes a rectangular shape; however, other embodiments may include a shape specific to an application. For example, the aperture 220 may include a round shape to accommodate an injector ram 222 having a round profile. Generally, the aperture 220 may be of any appropriate size, shape, and/or configuration.

The pig 30ⁱ may include a mechanism to minimize the amount of radiation passing out of the first chamber 162 via the aperture 220. For instance and as discussed above, an embodiment of the pig 30ⁱ may include a shutter 110 that is at least partially disposed over the aperture 220 and that is movable relative to the body 90 of the pig 30ⁱ. Generally, the shutter 110 may be movable between a first shutter position where it blocks the aperture 220, and a second shutter position where the shutter 110 no longer blocks the aperture 220 (e.g., when the ram 222 is extending through the aperture 220). In the second shutter position for the shutter, the aperture 220 may be characterized as being exposed to the interior of the pig 30ⁱ. As illustrated in Figures 4-6, the shutter 110 may be disposed over the aperture 220 during storage and transportation, and be displaced via the ram 222 or other mechanism that accesses the first chamber 162 to interact with the syringe 116 for a fluid discharge operation.

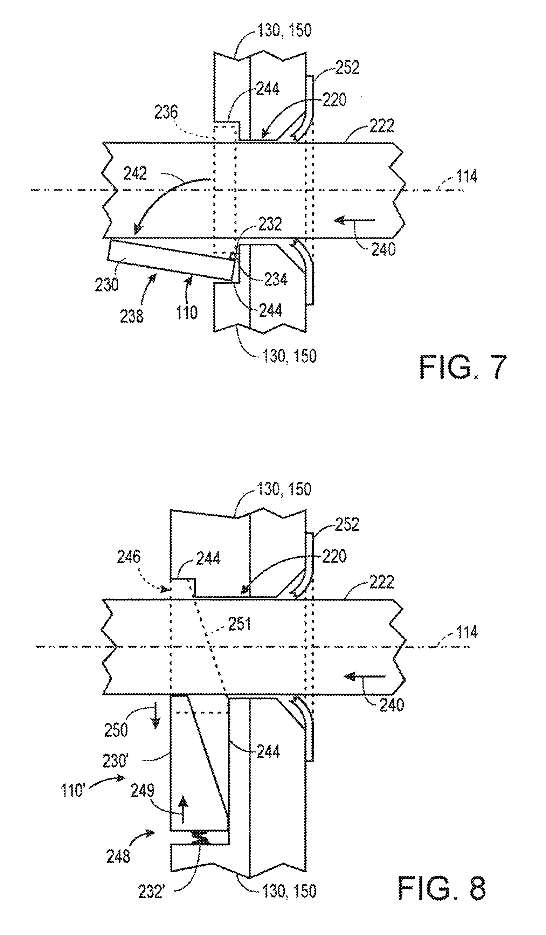

Turning to Figure 7, the shutter 110 may include a shutter body 230 and a biasing mechanism 232 that biases the shutter body 230 into position over the aperture 220 (e.g., biases the shutter 110 toward its first shutter position where it blocks the aperture 220). For example, Figure 7 illustrates an embodiment of the shutter 110 that includes the shutter body 230, the biasing mechanism 232, and a shutter pivot mechanism 234. As illustrated, the shutter body 230 may pivot or rotate between a closed position 236 (e.g., first shutter position) and an open position 238 (e.g., second shutter position). For example and in one embodiment, the biasing mechanism 232 may include a torsional spring that runs along the length of the shutter pivot mechanism 234. Thus, the biasing mechanism 232 may provide a biasing force to bias the shutter body 230 to the closed position 236, and to enable a force in the direction of arrow 240 (e.g., provided by movement of the ram 222 when engaged with the shutter 110), to pivot or rotate the shutter body 230 about the shutter pivot mechanism 234 in the direction of arrow 242 and to place the shutter 110 into the open position 238. Accordingly and when the ram 222 is retracted, the shutter body 230 may return to the closed position 236 via the biasing force provided by the biasing mechanism 232 (e.g., the torsional spring). The biasing mechanism 232 may be of any appropriate size, shape, configuration, and/or type, and may be incorporated in any appropriate manner. Multiple biasing mechanisms 232 may be utilized and disposed in any appropriate arrangement.

It should be noted that the shutter body 230 may be formed from or may otherwise incorporate a radiation-shielding material, such as lead or the like. For example, the body 230 may include shielding material throughout, or may include one or more layers of shielding material. In addition, the body 90 of the pig 30ⁱ and the shutter 110 may include features to prevent or reduce the likelihood of radiation passing out of the body 90 via the aperture 220. For example, the aperture 220 may include recesses 244 that accept the shutter body 230, and prevent or reduce the likelihood of radiation having a straight-line path via the aperture 220. In other words, the shutter body 230 may rest in the recesses 244 to overlap the edges of the first and second bodies 120 and 140 proximate to the aperture 220.

Figure 8 illustrates an alternate configuration of a shutter for a pig. Corresponding components between the embodiments of Figures 7 and 8 are identified by the same reference numeral. Those corresponding components that differ in at least some respect are identified by a "single prime" designation in Figure 8. The shutter 110' of Figure 8 is of a configuration to slide or axially move the shutter body 230' between a closed position 246 (e.g., a first shutter position) and an open position 248 (e.g., a second shutter position). For example and in one embodiment, the shutter body 230' may move orthogonally to the longitudinal axis 114 of the pig 30ⁱ between the closed position 246 and the open position 248. In the illustrated embodiment, the biasing mechanism 232' may be in the form of a compression spring that provides a biasing force in the direction of arrow 249, although any appropriate biasing mechanism or combination of biasing mechanisms may be used. Thus, the compressive spring may provide a biasing force to bias the shutter body 230' to the closed position 246, and to enable a force in the direction of arrow 240 to slide the shutter body 230' into the open position 248. Further, an angled taper 251 of the shutter body 230' may enable a force in the direction of arrow 240, (e.g., movement of the ram 222 in the direction of the arrow 240), to provide a force in the direction of arrow 240. Accordingly, inserting the ram 222 into the aperture 220 may move the shutter 110' into the open position 248 (e.g., by a camming action between the ram 222 and the shutter body 230'), and retracting the ram 222 may return the shutter 110 to the closed position 246 (e.g., in response to a disengagement of the ram 222 with the shutter body 230', and further in response to the biasing force provided by the biasing mechanism 232'). It should be noted that the movement of the shutter 110' may occur in a single plane in the Figure 8 configuration. This motion of the shutter 110' may be characterized as a sliding motion, an axial motion, or both.

Similar to the discussion regarding Figure 7, the shutter body 230' in the Figure 8 configuration may be formed from or incorporate a radiation-shielded material (e.g., lead). For example, the body 230' may include shielding material throughout, or may include a layer of shielding material. In addition, the body 90 and the shutter 110' may include features to prevent or reduce the likelihood of radiation passing out of the body 90 via the aperture 220. For example, the aperture 220 may include the recesses 244 that accept the shutter body 230', and prevent or reduce the likelihood of the radiation having a straight-line path via the aperture 220. In other words, the shutter body 230' may rest in the recesses 244 to overlap the edges of the first and second bodies 120 and 140 near the aperture 220.

It will also be appreciated that the shutters 110,110' as discussed in reference to Figures 4-8, may be retrofit to an existing radiation-shielded containment (e.g., a pig). For example, where a shielded containment includes or is adapted to include an aperture 220, the shutter 110/110' may be incorporated by an assembly that is mated directly to or proximate to the aperture 220. In other words, the shutter 110/110' may be part of an assembly that is inserted into a radiological container and disposed adjacent to an aperture to provide a shutter mechanism to close and open the aperture. For example, the shutter 110/110' may include a surrounding body that supports the shutter body 230/230' and the biasing mechanism 232/232'. Accordingly, the surrounding body may be coupled (e.g., threaded, affixed via an adhesive, and/or press fit) proximate to the existing aperture 220 to provide a shutter 110/110'.

Embodiments of the pig 30ⁱ may also include an aperture-sealing label 252 that may provide information, as well as a visual indication that a ram 222 of an injection device has passed through the aperture 220. For example and as illustrated in Figures 4-8, the aperture-sealing label 252 may be in the form of an adhesive label disposed on the exterior of the pig 30ⁱ (e.g., on the first and second body portions 100 and 102) such that passing the ram 222 through the aperture 220 may rupture, puncture, or perforate the aperture-sealing label 252. Figures 4 and 5 illustrate the aperture-sealing label 252 disposed over the aperture 220 prior to insertion of the ram 222 into the aperture 220. Figures 6-8 illustrate rupturing or puncturing the aperture-sealing label 252 via insertion of the ram 222 into the aperture 220. Accordingly, rupturing or puncturing the aperture-sealing label 252 may both provide a visual and physical indication that the ram 222 has at least attempted to engage the syringe 116 within the pig 30ⁱ. In other words, instead of opening the pig 30ⁱ to determine if the radiopharmaceutical has been dispensed, a clinician may simply visually inspect the aperture-sealing label 252 to determine if the syringe 116 has been engaged by the ram 222 (e.g., as evidenced by a rupturing of the label 252). Unlike the sealing label 158 previously described that may only indicate that the pig 30ⁱ has been opened, a hole-like puncture or the like in the aperture sealing label 252 may provide a positive indication that the ram 222 (or some other structure) has at least passed into the pig 30ⁱ (e.g., in an attempt to administer a radiopharmaceutical).

Other embodiments of the aperture-sealing label 252 may provide features beneficial to the design and operation of the pig 30ⁱ. For example and in one embodiment, the aperture-sealing label 252 may include or incorporate a radiation shielding material. In such an embodiment, the radiation shielding may prevent or reduce the likelihood of radiation escaping the body 90 via the aperture 220. Other embodiments may store information on the aperture-sealing label 252. For example, the sealing label 252 may include patient identification information, radiopharmaceutical calibration information, warnings, instructions for use, a serial number, an identification number, or the like. The sealing label 252 may be in the form of a bar code, an RFID tag, or the like. Accordingly and in one embodiment a complementary RFID device may be positioned proximate to the aperture-sealing label 252 to read the RFID information prior to injection of the radiopharmaceutical. For example, the ram 222 may include an RFID sensing device that reads the RFID information stored on the label 252 as the ram 222 is passed into the aperture 220. This may be beneficial to provide verification that the radiopharmaceutical is the correct type and dosage for injection into the respective subject. It should also be noted that similar embodiments may include placing a label with RFID information internal to the pig 30ⁱ (e.g., in the first chamber 162) such that the ram 222 may read the RFID information after the ram 222 has been inserted into the pig 30ⁱ (via the aperture 220).

It should be appreciated that in the above-described embodiments, the pig 30ⁱ includes a single syringe 116 that may be used to transport a single substance or a plurality of substances. In an embodiment with a syringe 116 that includes a radiopharmaceutical, it may be desirable that the entire amount of the radiopharmaceutical be flushed through the tube 118 and into the subject. For instance, the high cost of radiopharmaceuticals may make it desirable that all of the usable radiopharmaceutical be delivered into the subject, as opposed to having radiopharmaceutical being left in the tube 118 after each injection. Accordingly, embodiments of the syringe 116 may include a sequential syringe that is capable of housing and injecting two or more separate medical fluids. For example, the syringe 116 may include a pre-filled syringe having a first volume of fluid and a second volume of fluid that may be injected sequentially. Thus, certain embodiments may include advancing the plunger 174 to expel the first volume through the outlet 176 and continuing to advance the plunger 174 to eject the second volume via the outlet 176. For example, in the case of a radiopharmaceutical injection, the first volume may include a radiopharmaceutical and the second volume may include an inert solution (e.g., saline). Accordingly, continuing to advance the plunger 174 may flush the saline solution through the tubing 118 to ensure substantially all of the radiopharmaceutical is forced through the tube 118 and into the subject.

Figure 4 shows that a valve 177 may be disposed with the tube 118 (e.g., adjacent to the syringe 116), or may be incorporated into the outlet 176 of the syringe 116. This valve 177 may be of any appropriate type (e.g., a one-way or check valve). In one embodiment, the valve 177 is used to retain a radioactive fluid in the syringe 116, while the entirety of the tube 118 "downstream" of the valve 177 is devoid of any fluid that is intended for injection into a patient (e.g., this portion of the tube 118 is "empty" in this instance). In another embodiment, the potion of the tube118 downstream of this valve 177 includes a medical fluid (e.g., saline) that is of a different type than what is contained within the syringe 116. The end of the tube118 could incorporate an appropriate fluid seal (for either instance). Generally, the valve 177 may be used to maintain a separated state between the syringe 116 and the portion of the tube 118 downstream of the valve 177 until a fluid delivery operation is initiated. Advancement of the syringe plunger 176 (e.g., via a corresponding advancement of the ram 222) may open this valve 177 to allow fluid from the syringe 116 to flow into the tube 118. If the tube 118 initially contains a fluid, this initial advancement of the plunger 176 should result in the fluid from the tube 118 being delivered, followed by fluid from the syringe 116. There may of course be some mixing of the two fluids at the interface.

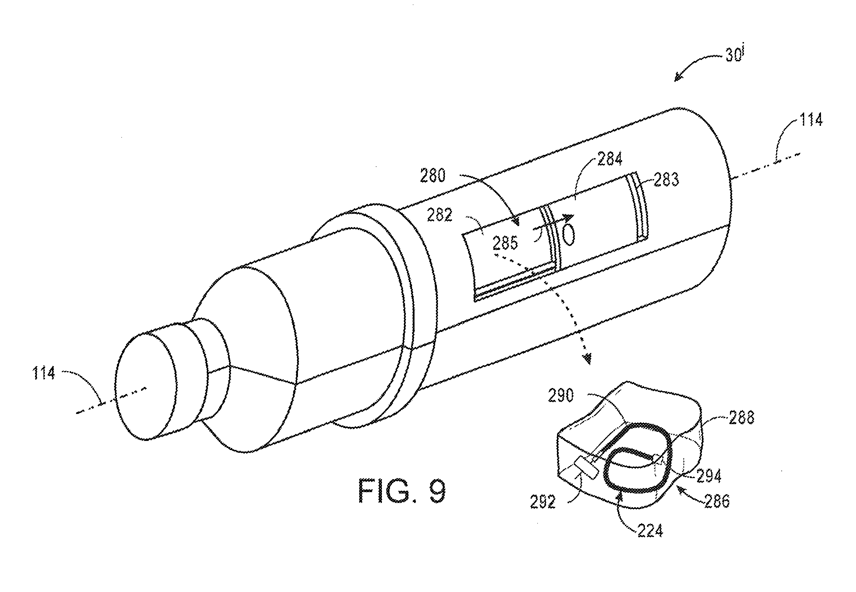

Figure 9 illustrates that the pig 30ⁱ may include a compartment 280 that is capable of storing additional devices and medical equipment, and which may be isolated from each of the first chamber 162 and the second chamber 164. For example, the first body section 100 may include the compartment 280 having a first recess 282, a second recess 283, and an access or door 284. The first recess 282 may include a cavity in the first body portion 120 that is capable of housing medical devices 286. The second recess 283 may include a region capable of accepting the door 284 when it is moved in the direction of arrow 285. Accordingly, the compartment 280 may house a variety of medical equipment that may be used in the process of injecting a subject. For example and in the illustrated embodiment, the medical device 286 may include injection peripherals (e.g., a patient connection set 224) having a sealed package 288 and including a patient line tube 290, a catheter/needle 292, and a connector 294. As will be appreciated, the patient connection set 224 may be coupled to the connector 214 of the tubing 118 (e.g., Figure 6) after being removed from the sealed package 288. Accordingly, the pig 30ⁱ may be pre-loaded with medical devices that may be used by a clinician during the administration of a radiopharmaceutical. For example, instead of locating and retrieving a patient connection set 224 to mate with the tube 118, the clinician may simply retrieve the needed medical devices 286 from the compartment 280 (e.g., before or after installing the pig 30¹ on a power injector). In other embodiments, the medical devices may include connectors, tubing, needles, valves, sterilization pads, tape, catheters, and the like.

As previously discussed, the pig 30ⁱ may be employed in a variety of nuclear medicine applications, including the administration of a radiopharmaceutical. For example, the pig 30ⁱ discussed above may be employed to inject a patient with a radiopharmaceutical that may enable an imaging system to acquire data related to the injection and process the data to diagnose a patient's condition. However, the pig 30ⁱ may contain any appropriate radioactive fluid and which may provide any appropriate function or combination of functions when injected into a patient.

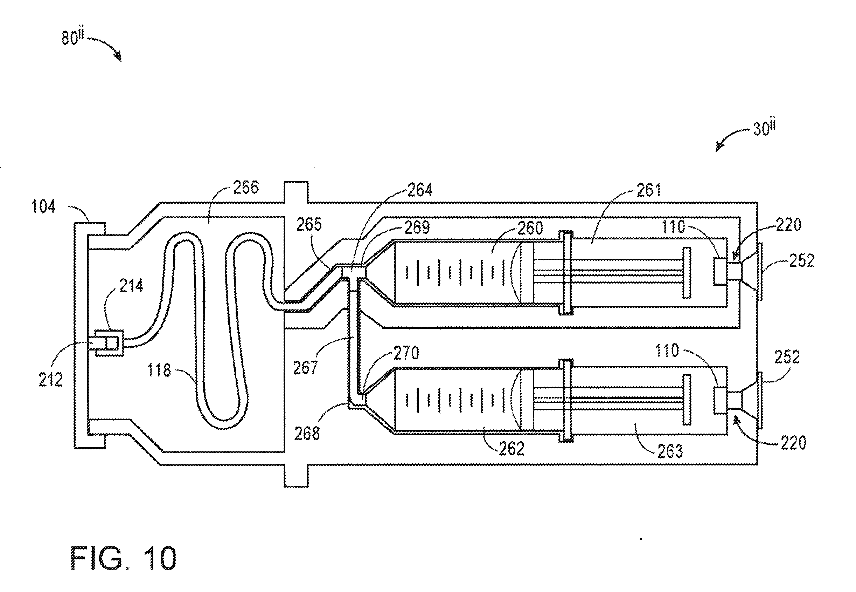

Turning now to Figure 10, another embodiment of a radiological containment or pig is illustrated that accommodates multiple syringes. Corresponding components between the embodiments of Figures 2-6 and Figure 10 may be identified by the same reference numeral. Those corresponding components that are identified by the same reference numeral and that differ in a manner that is addressed herein, are identified by a superscripted "ii" designation. Therefore, the pig in Figure 10 is identified by reference numeral 30ⁱⁱ.

Generally, the pig 30ⁱⁱ from Figure 10, a first syringe 260, a second syringe 262, and a tube 118 may be characterized as a fluid delivery system 80ⁱⁱ. More specifically, the illustrated embodiment includes a first syringe 260 disposed in a first shielded chamber 261 of the pig 30ⁱⁱ, a second syringe 262 disposed in a second chamber 263 of the pig 30ⁱⁱ, a valve 264 disposed in a first passage 265 of the pig 30ⁱⁱ, a tube 118 extending from the valve 264, through the first passage 265, and into a third chamber 266 of the pig 30ⁱⁱ, and a second tube 267 that extends from the second syringe 262, through a second aperture 268 of the pig 30ⁱⁱ, and to the valve 264. The valve 264 may selectively enable fluid to flow into the tube 118 from an outlet 269 of the first syringe 260, or from the second tube 267 in fluid communication with an outlet 270 of the second syringe 262. In an embodiment that includes transportation and delivery of a radiopharmaceutical, the first syringe 260 may be pre-filled with a radiopharmaceutical, whereas the second syringe 262 (as well as possibly the second tube 267, or each of the second tube 267 and tube 118) and may be pre-filled with a saline solution. Accordingly, the injection of the radiopharmaceutical may include coupling the tube 118 to the subject, and advancing the plunger of the first syringe 260 to flush the saline solution through the tube 118 and to thereafter inject the radiopharmaceutical from the first syringe 260 via the tube 118. Subsequently, the plunger of the second syringe 262 may be advanced to force the saline solution of the second syringe 262 into the tube 118 via the second tube 267 and the valve 264. The injection of the saline solution from the second syringe 262 may ensure that the radiopharmaceutical is flushed out of the tube 118 and into the subject. Notwithstanding the foregoing, any appropriate fluid may be contained in each of the syringes 260, 262, and the syringes 260, 262 may be operated in any sequence for a fluid delivery operation. Moreover, each of the syringes 260, 262 may be in accordance with the syringe 116 discussed above.

As will be appreciated, embodiments of the fluid delivery system 80ⁱⁱ including dual syringes may include features similar to those described above with regard to the embodiment of Figures 2-6. For example, the first chamber 261 may be appropriately shielded to isolate radiation from the surroundings and the third chamber 266 may house the tube 118. In certain embodiments, the second chamber 263 and/or the third chamber 266 may also incorporate an appropriate shielding. Further, embodiments may include an aperture 220, aperture sealing labels 252, shutters 110/110', a cap 104, and the like. It should also be noted that in certain embodiments, the second chamber 263 may not require shielding because it merely houses a saline or other inert solution that does not emit radiation into the surrounding environment. Other embodiments may include any number of cavities internal to the pig 30ⁱⁱ that may house any number of medical containers (e.g., syringes 116)

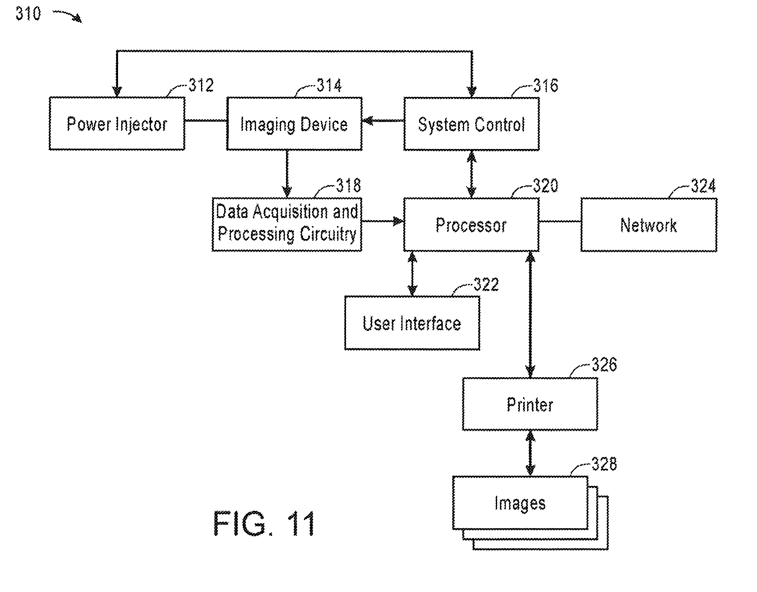

Referring now to the block diagram of Figure 11, an imaging system 310 may include a power injector 312 adapted to inject medical fluids from a syringe (and/or a plurality of syringes) installed on power injector 312. For example, the medical fluids may include a drug, a contrast agent, a radiopharmaceutical, a tagging agent, a saline solution, or a combination thereof. Imaging system 310 further includes an imaging device 314, a system control 316, data acquisition and processing circuitry 318, a processor 320, a user interface 322, and a network 324 (or any appropriate communications link). Specifically, the imaging device 314 is configured to obtain signals representative of an image of a subject after the medical fluid (e.g., contrast agent or radiopharmaceutical) has been administered to the subject via the power injector 312. The imaging system 310 may include a positron emission tomography (PET) system, a single photon emission computer tomography system (SPECT), a nuclear medicine gamma ray camera, a magnetic resonance imaging (MRI) system, or another suitable imaging modality. Image data indicative of regions of interest in a subject may be created by the imaging device 314, either in a conventional support, such as photographic film, or in a digital medium.

The system control 316 may include a wide range of circuits, such as imaging (e.g., radiation) source control circuits, timing circuits, circuits for coordinating data acquisition in conjunction with patient or table movements, circuits for controlling the position of imaging (e.g., radiation) detectors, and so forth. The imaging device 314, following acquisition of the image data or signals, may process the signals, such as for conversion to digital values, and forward the image data to data acquisition circuitry 318. In the case of analog media, such as photographic film, the data acquisition system 318 may generally include supports for the film, as well as equipment for developing the film and producing hard copies that may be subsequently digitized. For digital systems, the data acquisition circuitry 318 may perform a wide range of initial processing functions, such as adjustment of digital dynamic ranges, smoothing or sharpening of data, as well as compiling of data streams and files, where desired. The data is then transferred to the processor 320 where additional processing and analysis may be performed. For conventional media such as photographic film, the processor 320 may apply textual information to films, as well as attach certain notes or patient-identifying information. In a digital imaging system, the data processing circuitry of the processor 320 may perform substantial analyses of data, ordering of data, sharpening, smoothing, feature recognition, and so forth.

Ultimately, the image data may be forwarded to an operator/user interface 322 for viewing and analysis. While operations may be performed on the image data prior to viewing, the operator interface 322 is at some point useful for viewing reconstructed images based upon the image data collected. In the case of photographic film, images may be posted on light boxes or similar displays to permit radiologists and attending physicians to more easily read and annotate image sequences. The image data can also be transferred to remote locations, such as via the network 324. In addition, the operator interface 322 may enable control of the imaging system 310 (e.g., by interfacing with the system control 316). Furthermore, the imaging system 310 may include a printer 326 to output a hard copy of images 328.

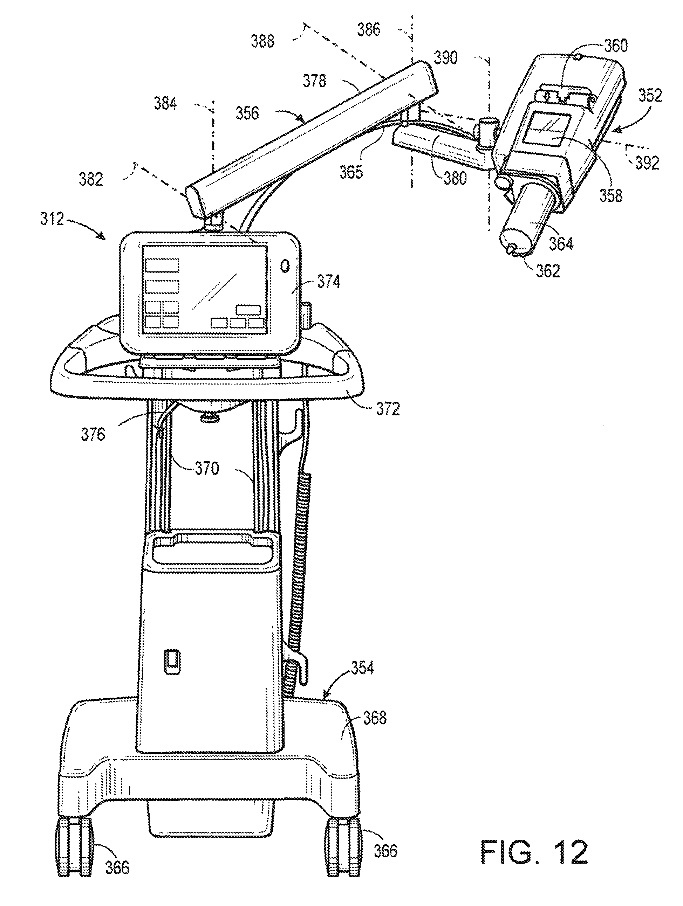

Figure 12 is a perspective view of one embodiment for the power injector 312 shown in Figure 11, and which may be used to discharge fluid from a containment 364 (e.g., pig 30ⁱ/30ⁱⁱ) when installed thereon. This power injector 312 may be considered to be part of the above-discussed fluid delivery systems 80ⁱ/80ⁱⁱ. In this regard, the power injector 312 may be described for the case where the containment 364 is in the form of pig 30ⁱ.

The power injector 312 in Figure 12 may include a powerhead 352, a stand assembly 354, and a support arm 356. Powerhead 352 may include a mounting system adapted to aptly capture the pig 30ⁱ with the syringe 116 and tube 118 installed therein. For example, such a mounting system may realize desired fluid injection parameters, such as pressure provided by the ram of the powerhead 352 (e.g., ram 222) to the syringe plunger 174, for injecting fluid at a desired rate. The mounting system included with powerhead 352 may also be adapted to simplify tasks associated with installing the pig 30ⁱ on the powerhead 352, which may further enable the clinician to attend to additional tasks associated with other aspects of an injection procedure. For example, the pig 30ⁱ may be installed directly on the powerhead 352 without removing the syringe 116 from the pig 30ⁱ. During the injection, the ram of the powerhead 352 (e.g., ram 222) may engage or otherwise interact with the plunger 174 of the syringe 116 via the aperture 220 in the pig 30ⁱ. Powerhead 352 may further include a display 358, a fluid control bar 360, and an air detector 362. Fluid control bar 360 may facilitate manual manipulation of the plunger 174 in the pig 30ⁱ when installed on the powerhead 352. Air detector 362 may signal a controller, such as controller 316 (Figure 11) when air is detected in or leaving the pig 30ⁱ.

The illustrated stand assembly 354 includes a set of four wheels 366, a chassis 368, vertical supports 370, a handle 372, and a display 374. The vertical supports 370 may adjustably elevate handle 372, display 374, and support arm 356 above chassis 368, and, in certain embodiments, it may have a recessed portion through which power cable 376 is routed. Display 374 may include a liquid crystal display, a cathode ray tube display, an organic light emitting diode display, a surface emission display, or other appropriate display. Support arm 356 of injector 312 shown in Figure 12 includes multiaxis articulating members 378, 380. The illustrated articulating member 378 has two degrees of freedom relative to chassis 368 due to two perpendicular axes of rotation 382, 384. Similarly, the articulating member 380 has two degrees of freedom relative to articulating member 378 by virtue of two perpendicular axes of rotation 386, 388. Power cable 376 is shown as being routed along the articulating members 378, 380 to powerhead 352.

Powerhead 352 of Figure 12 may couple to articulating member 380 via a joint that provides two degrees of freedom relative to articulating member 380. As a result, the powerhead 352 may rotate about axes 390, 392. In total, the illustrated powerhead 352 has six degrees of freedom relative to the chassis 368. Other embodiments may include more or fewer degrees of freedom. It should be appreciated that any power injector used by the fluid delivery systems 80ⁱ/80ⁱⁱ may be of any appropriate size, shape, configuration, and/or type.

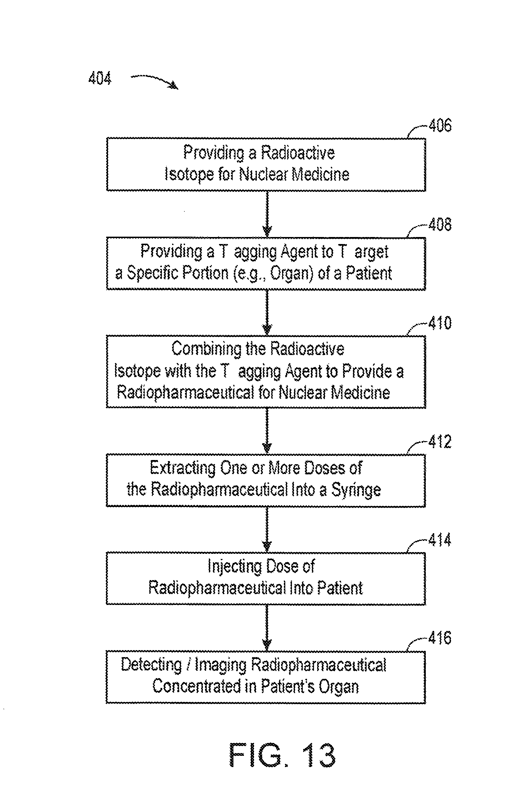

Figure 13 is a flowchart illustrating an exemplary nuclear medicine process or protocol utilizing the power injector 312 illustrated with reference to Figures 11 and 12. As illustrated, protocol 404 begins by providing a radioactive isotope for nuclear medicine at block 406. For example, block 406 may include, for example, eluting technetium-99m from a radioisotope generator. At block 408, protocol 404 proceeds by providing a tagging agent (e.g., an epitope or other appropriate biological directing moiety) adapted to target the radioisotope for a specific portion (e.g., an organ) of a patient. At block 410, protocol 404 then proceeds by combining the radioactive isotope with the tagging agent to provide a radiopharmaceutical for nuclear medicine. In certain embodiments, the radioactive isotope may have natural tendencies to concentrate toward a particular organ or tissue and, thus, the radioactive isotope may be characterized as a radiopharmaceutical without adding any supplemental tagging agent. At block 412, protocol 404 then may proceed by extracting one or more doses of the radiopharmaceutical into a syringe or another container, such as a container suitable for administering the radiopharmaceutical to a patient in a nuclear medicine facility or hospital. At block 414, the protocol 404 proceeds by injecting or generally administering a dose of the radiopharmaceutical into a patient. After a pre-selected time, the protocol 404 proceeds by detecting/imaging the radiopharmaceutical tagged to the patient's organ or tissue (block 416). For example, block 416 may include using a gamma camera or other radiographic imaging device to detect the radiopharmaceutical disposed on or in or bound to tissue of a brain, a heart, a liver, a tumor, a cancerous tissue, or various other organs or diseased tissue.

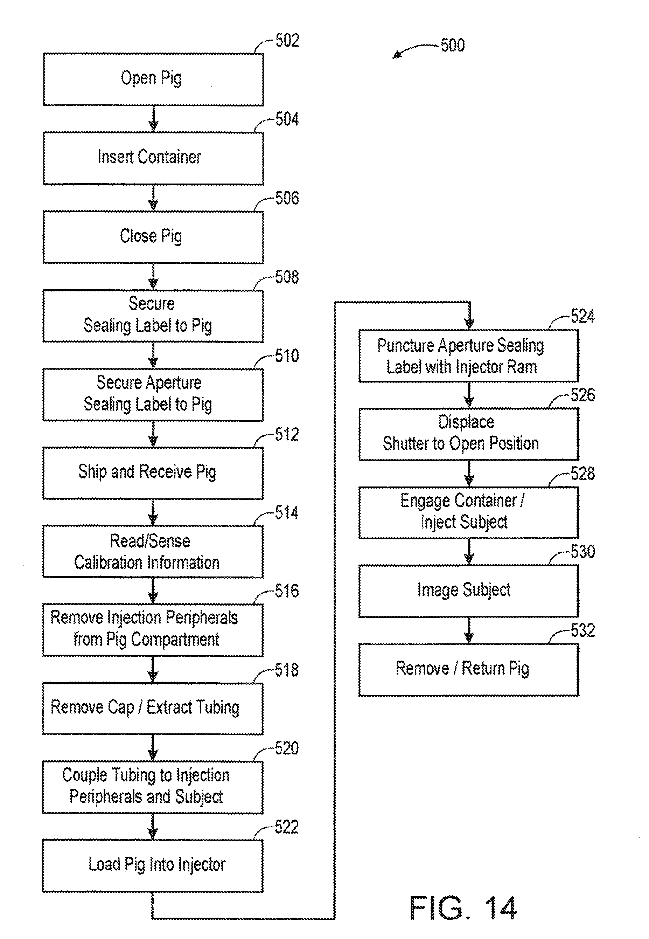

In accordance with a variety of embodiments disclosed above, the injection process may include procedures that reduce the potential exposure of a clinician to radiation. For example and as illustrated in Figure 14, an exemplary injection protocol 500 may include opening the pig (block 502), inserting a container (e.g., a syringe) into the pig (block 504), closing the pig (block 506), securing a sealing label to the pig (block 508), securing an aperture sealing label to the pig (block 510), shipping and receiving the pig (block 512), reading or sensing calibration information (block 514), removing injection peripherals from a compartment in the pig (block 516), removing a cap to extract the tubing (block 518), coupling tubing to the injection peripherals and coupling the injection peripherals to a subject (block 520), loading the pig into an injector (block 522), advancing a ram of the injector to puncture the aperture sealing label with the injector ram (block 524), displacing a shutter to an open position (block 526), advancing the ram of the injector to engage or otherwise interact with the container (e.g., syringe plunger) and inject the subject (block 528) with the contents of the container, imaging the subject (block 530), and, finally, retracting the injector ram and removing the pig for return to the supplier facility (block 532). Accordingly, the injection and imaging process may not include opening the pig to remove a syringe therefrom to do the injection and, thus, may reduce the potential for exposing a clinician to radiation. As will be appreciated, each of these steps may be varied in detail and sequence to provide injections in specific applications and system setups. For example, reading and sensing calibration information (block 514) may be conducted proximate to the time the ram punctures the aperture-sealing label. Further, each of the steps may include details discussed with regard to Figures 1-13.

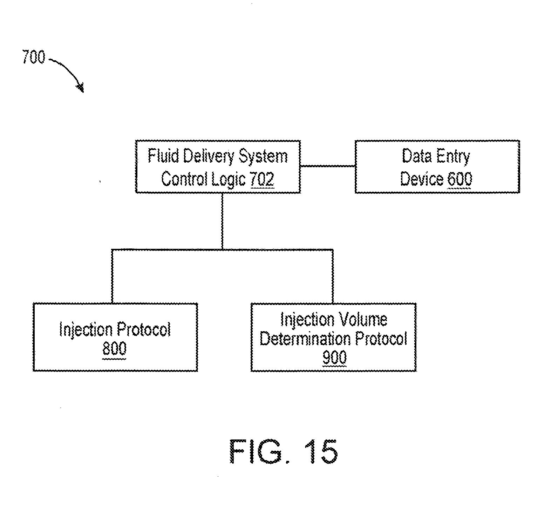

Figure 15 presents a schematic of one embodiment of a fluid delivery system 700 (e.g., the fluid delivery systems 80ⁱ, 80ⁱⁱ, and which as discussed above may be in the form of a pig installed on a power injector, where the pig houses at least one syringe and tubing, and where a ram may interact with the syringe plunger). The fluid delivery system 700 may include one or more data entry devices 600 of any appropriate configuration and/or type. A given data entry device 600 (e.g., a keyboard, a mouse, a touch pad, a track ball, a touch screen display, a soft key display) may allow an operator to provide information to fluid delivery system control logic 702. A given data entry device 600 may be an appropriate reader (e.g., an RFID reader or the like) to read or retrieve information that has been stored in any appropriate manner (e.g., information stored on or in relation to a pig 30ⁱ/30ⁱⁱ).

The fluid delivery system control logic 702 of the fluid delivery system 700 may be of any appropriate form and/or configuration, may be implemented or integrated in any appropriate manner, or both (e.g., for instance in the power injector software; implemented by software, hardware, firmware, and any combination thereof). The functionality of the control logic 702 may be provided by one or more processors of any appropriate size, shape, configuration, and/or type. The functionality of the control logic 702 may be provided by one or more computers of any appropriate size, shape, configuration, and/or type. At least one graphical user interface (e.g., display 358 in Figure 12) may be operatively interconnected with the fluid delivery system control logic 702 to present an appropriate output (e.g., to an operator of the corresponding power injector). The fluid delivery system control logic 702 may also be operatively interconnected with a syringe plunger driver comprising a ram and motorized drive source (e.g., ram 222 discussed above).

The fluid delivery system control logic 702 may be configured to include at least one fluid delivery or injection protocol 800 (e.g., for a medical application, and which may be referred to as a medical fluid delivery procedure or operation) and a delivery or injection volume determination protocol 900, and each of which may be in the form of a programmed sequence. Each injection protocol 800 may be configured to control the manner in which one or more fluids are being delivered to a fluid target, such as by being injected into a patient. A particular fluid delivery protocol 800 may be configured to deliver a programmed volume of a first fluid at a programmed flow rate, as well as a programmed volume of a second fluid at a programmed flow rate. Each delivery of each of the first and second fluids may be characterized as a phase. In one embodiment, the first fluid is contrast media and the second fluid is saline. Generally, the injection protocol 800 may be configured to include one or more phases for at least one fluid. The injection volume determination protocol 900 will be discussed in more detail below, but generally is configured to determine the volume of a unit dose necessary to provide a prescribed dose of a radiopharmaceutical, based on the real-time radioactivity level of the unit dose.

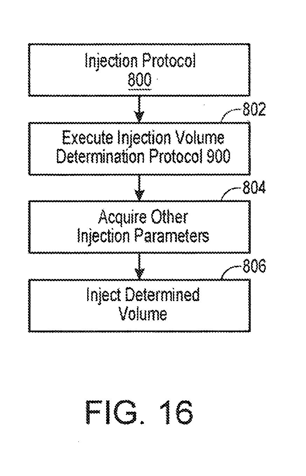

With reference to Figure 16, one embodiment of the injection protocol 800 is illustrated. In step 802, the injection volume determination protocol 900 is executed. Although step 802 will be discussed in more detail below, the injection volume determination protocol 900 is generally configured to determine what volume of a unit dose is required to provide a prescribed dose of a radiopharmaceutical (an injection volume), based on the real-time radioactivity level of the unit dose. Because the radioactive magnitude or level of a radiopharmaceutical decreases over time, the protocol 900 provides healthcare practitioners with an accurate indication of the appropriate volume of the unit dose to administer to the patient to provide for effective treatment of the patient. Once the correct volume of the unit dose to administer to the patient is determined (e.g., the injection volume) or beforehand, other injection parameters may be acquired in step 804, and which may be executed in any appropriate manner (i.e., steps 802 and 804 may be executed in any order). Examples of these "other" injection parameters, include type of fluid to inject, length of time for injection, flowrate, a flowrate suspension or termination condition, and the like. After the injection volume has been determined (e.g., the portion of the unit dose to be injected to provide the prescribed dose), along with the other injection parameters, the determined volume is injected pursuant to step 806.

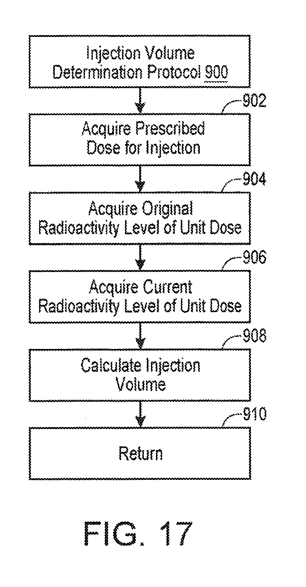

Referring to Figure 17, one embodiment of the injection volume determination protocol 900 is shown. As previously mentioned, the protocol 900 permits a healthcare practitioner or user to administer an accurate dose of the radiopharmaceutical or other radioactive fluid. Broadly, the protocol 900 allows a user to specify a radioactive dose to be injected or administered instead of programming a specific volume of the radiopharmaceutical. For instance, in step 902, the user can specify a value for a radioactive dose variable used by the protocol 900 (e.g., the value being a prescribed dose or desired dose to be injected into a patient) of the radiopharmaceutical in terms of the radioactivity or magnitude of. the prescribed dose or desired dose (e.g., 25 mCi)). A value for the radioactive dose variable is enterable to the injection volume determination protocol 900 in any appropriate manner (e.g., more generally to the fluid delivery system 700 of Figure 15, and which may be used by the power injector 312 of Figure 12). For example, a user could input a value for a radioactive dose variable via the data entry device 600 of Figure 15 (e.g., via a graphical user interface; by an operator using an appropriate reader to retrieve stored information) or this value could be automatically retrieved (e.g., by a reader incorporated by a power injector, and which may read this value from a pig installed on the power injector).

Step 904 of the injection volume determination protocol 900 entails acquiring the original radioactivity level of a unit dose (e.g., 25 mCi), and which may be used for an original radioactively level variable used by the protocol 900. The unit dose may be characterized as the quantity of radioactive fluid that is originally loaded into a syringe (e.g., syringe 116), and which may be housed in a radiological containment (e.g., pig 30ⁱ). That is, the unit dose may be viewed as the "bulk" source to be used for an injection. In any case, other information regarding the unit dose may be acquired through execution of step 904, for instance the chemical make-up of the unit dose, the time and date that the original radioactivity level was acquired, the location where the unit dose was created, the volume of the unit dose, the approximate number of atoms of the unit dose, the half-life of the unit dose, the decay constant for the unit dose, and the like. The original radioactivity level and other information associated with the unit dose may be referred to as "unit dose information."

In one embodiment, the unit dose referenced in relation to step 904 of the injection volume determination protocol 900 is created or filled at a first location (e.g., laboratory) and then transported to a second location (e.g., hospital) where the injection protocol 800 and the injection volume determination protocol 900 are to be executed. However, the protocol 900 contemplates that the unit dose could be created at the same location that the protocols 800 and 900 are to be executed. Regardless, there will very likely be some length of time between the time the unit dose is created and the time the protocols 800 and 900 are to be executed. This length of time may be utilized by step 906 of the injection volume determination protocol 900 (described infra) to calculate the current radioactivity level of the unit dose.

Step 904 may acquire the unit dose information in any appropriate manner. In one embodiment, a containment housing the unit dose (e.g., pig 30ⁱ) is encoded or provided with the unit dose information, preferably at the time the unit dose is created or filled. For instance, the sealing label 252 of the pig 30ⁱ could be encoded or provided with the unit dose information to be read prior to injection of the radiopharmaceutical, and utilized by the protocols 800 and 900 to inject or administer an appropriate dose to a patient. In one variation, the sealing label 252 of the pig 30ⁱ may include the unit dose information on an RFID tag or the like, and this information could be read by an RFID sensing device on the ram of the syringe plunger driver (e.g., ram 222) prior to injection of the radiopharmaceutical. Any appropriate reader could be utilized. Also, a sealing label 158 on a pig 30ⁱ could be encoded or provided with the unit dose information to be read by the user, any appropriate sensing/reading device, or the like. Instead of RFID tags and a reader antenna, other information storing and sensing devices and means could be utilized such as bar codes, serial number, identification numbers, and the like. Further, alternative to sealing labels for providing the user or healthcare practitioner with the unit dose information, the protocol 900 envisages other means such as a verified certificate or other documentation accompanying the unit dose to be read by the user or a sensing device, information provided on a syringe, or the like. Generally, the unit dose information may be acquired in any appropriate manner for purposes of step 904 of the protocol 900.

The current radioactivity level of the unit dose is acquired in step 906 of the injection volume determination protocol 900. For instance, the protocol 900 may determine the quantity of time elapsed since the original radioactivity level of the unit dose was acquired, such as for example, by reading or sensing the unit dose information on the sealing label or other documentation as previously described. Thereafter, the protocol 900 may provide for the execution of a number of calculations. For instance, the protocol 900 may obtain a first calculation in the form of taking the base log to the negative power of the decay constant of the unit dose, multiplied by the time elapsed since the original radioactivity level of the unit dose was acquired. Next, the protocol 900 may provide for the execution of a second calculation in the form of multiplying the original radioactivity level by the first calculation. Finally, the protocol 900 may obtain a real-time determination of the radioactivity of the unit dose by dividing the second calculation by the volume of the radioactive fluid in the unit dose. While one method has been described to acquire the current radioactivity level of the unit dose, it should be appreciated that various other methods and devices can be utilized without departing from the spirit and scope of the protocol 900. In one embodiment, the current radioactivity level of the unit dose is acquired through execution of step 906 without removing a syringe (e.g., syringe 116) from a radiological containment (e.g., pig 30ⁱ), where the syringe contains a radiopharmaceutical or other radioactive fluid/material.

Once the unit dose current radioactivity level is acquired pursuant to step 906 of the injection volume determination protocol 900, the protocol 900 calculates the injection volume of the unit dose that should provide the prescribed dose, all through execution of step 908. For instance, the prescribed dose (step 902) may be divided by the real-time unit dose radioactivity level (step 906) in order to obtain the required injection volume. Once step 908 calculates the injection volume of the unit dose that should provide the prescribed dose, the injection volume determination protocol 900 returns to the injection protocol 800 via execution of step 910. However, the protocol 900 also contemplates that the injection volume determination protocol 900 could be utilized by a healthcare practitioner apart from the injection protocol 800. For instance, the practitioner may desire to know a real-time determination of a unit dose volume to produce some "prescribed" dose radioactivity level for running experiments or other studies, calibrating the control system or power injector, or the like.

While the invention may be susceptible to various modifications and alternative forms, specific embodiments have been shown by way of example in the drawings and have been described in detail herein. However, it should be understood that the invention is not intended to be limited to the particular forms disclosed. Rather, the invention is to cover all modifications, equivalents, and alternatives falling within the scope of the invention as defined by the following appended claims.

## Claims

1. A fluid delivery system (80'), comprising:
a containment (30') comprising a body (90), wherein said body comprises first and second chambers (162, 164) and a passage (190) that extends between said first and second chambers;
a first syringe (116) disposed within said first chamber (62); and
medical tubing (118) comprising first and second conduit sections (118a, 118b), wherein said first conduit section (118a) is interconnected with said first syringe (116), wherein said medical tubing (118) extends through said passage (190), and wherein said second conduit section (118b) is disposed within said second chamber (164) and is fluidly interconnected with said first conduit section (118a),
said medical tubing (118) being adapted for extraction out of said containment (30') for a fluid delivery operation whilst remaining connected to said syringe (116).

2. The fluid delivery system of claim 1, further comprising a cap (104) detachably interconnected with said body of said containment.

3. The fluid delivery system of claim 2, wherein one end of said second conduit section (118b) is removably coupled to said cap (104).

4. The fluid delivery system of claim 3, wherein said cap (104) comprises a protrusion (212) that extends within an open end of said second conduit section (118b).

5. The fluid delivery system of claim 3 or claim 4, wherein said second conduit section (118b) engages a surface of said cap (104) that interfaces with said second chamber (164) when said cap is mounted on said body (90) of said containment (30').

6. The fluid delivery system of any preceding claim, further comprising radiological shielding (130) for said first chamber (162).

7. The fluid delivery system of any preceding claim, further comprising a valve (177) disposed within said medical tubing (118).

8. The fluid delivery system of claim 7, wherein said medical tubing (118) contains a first fluid, and wherein said first syringe (116) contains a second fluid.

9. The fluid delivery system of claim 8, wherein said first fluid comprises saline and wherein said second fluid comprises a radioactive fluid.

10. The fluid delivery system of any preceding claim, wherein said body (90) of said containment (30') comprises a necked end portion (124) that coincides with an end of said second chamber (164).

11. The fluid delivery system of any preceding claim, wherein said passage (190) comprises a first passage section that extends from said first chamber (162) along a longitudinal axis of said containment, a second passage section that extends from said first passage section and away from said longitudinal axis, and a third passage section that extends parallel to but offset from said first passage section.

12. The fluid delivery system of claim 11, wherein said containment (30') further comprises second radiological shielding disposed about said passage (190).

13. The fluid delivery system of any preceding claim, wherein said containment (30') further comprises:
an access aperture (220) for said body (90),
a shutter (110) movable relative to said body between first and second shutter positions, wherein said shutter (110) blocks said access aperture when in said first shutter position, and wherein said shutter exposes said access aperture to an interior of said body when in said second shutter position; and
first shielding associated with said first chamber (162), wherein said first chamber is aligned with said access aperture, wherein said access aperture is adapted to accept a ram of an injection device for interacting with said plunger of said first syringe (116).

14. The fluid delivery system of claim 13, further comprising a biasing member that interacts with said shutter (110) to bias said shutter into said first shutter position.

15. The fluid delivery system of claim 14, wherein said shutter comprises radiological shielding.

16. The fluid delivery system of claim 13, further comprising a label (252) that blocks said access aperture (220) of said containment.

17. The fluid delivery system of claim 16, wherein said label (252) comprises radiological shielding.

18. The fluid delivery system of claim 17, wherein said label (252) is selected from the group consisting of a bar code, an RFID tag, or human readable label.

19. The fluid delivery system of any preceding claim, wherein said body (90) comprises first and second body sections that are interconnected by a hinge (160), and movable relative to each other between open and closed positions.

20. The fluid delivery system of any preceding claim, wherein said body (90) comprises a third chamber (263), wherein said fluid delivery system further comprises a second syringe (262) disposed within said third chamber (263).

21. The fluid delivery system of claim 20, wherein said first and second syringes discharge into a common conduit of said medical tubing.

## Patentansprüche

1. Ein Fluidbereitstellsystem (80'), das aufweist:
eine Verkapselung (30') mit einem Körper (90), wobei der Körper erste und zweite Kammer (162, 164) und einen Kanal (190) hat, der sich zwischen der ersten und der zweiten Kammer erstreckt,
eine erste Spritze (116), die sich in der ersten Kammer (62) befindet, und
eine medizinische Rohrleitung (118), die einen ersten und einen zweiten Leistungsabschnitt (118a, 118b) hat, wobei der erste Leitungsabschnitt (1 18a) mit der ersten Spritze (116) verbunden ist, wobei sich die medizinische Rohrleitung (118) durch den Kanal (190) erstreckt und wobei der zweite Leitungsabschnitt (118b) in der zweiten Kammer (164) angeordnet ist und mit dem ersten Leitungsabschnitt (118a) in Fluidverbindung steht,
wobei die medizinische Rohrleitung (118) zur Extraktion aus der Verkapselung (30') heraus für einen Fluidbereitstellvorgang angepasst ist, während eine Verbindung mit der Spritze (116) bleibt.

2. Das Fluidbereitstellsystem nach Anspruch 1, das ferner eine Kappe (104) aufweist, die mit dem Körper der Verkapselung lösbar verbunden ist.

3. Das Fluidbereitstellsystem nach Anspruch 2, wobei ein Ende des zweiten Leitungsabschnitts (118b) mit der Kappe (104) entfernbar gekoppelt ist.

4. Das Fluidbereitstellsystem nach Anspruch 3, wobei die Kappe (104) einen Vorsprung (212) aufweist, der mit einem offenen Ende des zweiten Leitungsabschnitts (118b) verbunden ist.

5. Das Fluidbereitstellsystem nach Anspruch 3 oder 4, wobei der zweite Leitungsabschnitt (118b) mit einer Fläche der Kappe (104) in Eingriff steht, die mit der zweiten Kammer (164) gekoppelt ist, wenn die Kappe an dem Körper (90) der Verkapselung (30') montiert ist.

6. Das Fluidbereitstellsystem nach einem der vorhergehenden Ansprüche, das ferner eine radiologische Abschirmung (130) für die erste Kammer (162) aufweist.

7. Das Fluidbereistellsystem nach einem der vorhergehenden Ansprüche, das ferner ein Ventil (177) aufweist, das sich in der medizinischen Rohrleitung (118) befindet.

8. Das Fluidbereitstellsystem nach Anspruch 7, wobei die medizinische Rohrleitung (118) ein erstes Fluid enthält und wobei die erste Spritze (116) ein zweites Fluid enthält.

9. Das Fluidbereitstellsystem nach Anspruch 8, wobei das erste Fluid eine physiologische Kochsalzlösung aufweist und das zweite Fluid ein radioaktives Fluid aufweist.

10. Das Fluidbereitstellsystem nach einem der vorhergehenden Ansprüche, wobei der Körper (90) der Verkapselung (30') einen verengten Endabschnitt (124) aufweist, der mit einem Ende der zweiten Kammer(164) zusammenfällt.

11. Das Fluidbereitstellsystem nach einem der vorhergehenden Ansprüche, wobei der Durchlass (190) einen ersten Durchlassabschnitt, der sich von der ersten Kammer (162) entlang einer Längsachse der Verkapselung erstreckt, einen zweiten Durchlassabschnitt, der sich von dem ersten Durchlassabschnitt und von der Längsachse weg erstreckt, und einen dritten Durchlassabschnitt, der sich parallel zum ersten Durchlassabschnitt jedoch versetzt von diesem erstreckt, aufweist.

12. Das Fluidbereitstellsystem nach Anspruch 11, wobei die Verkapselung (30') ferner eine zweite radiologische Abschirmung aufweist, die sich um den Durchlass (190) befindet.

13. Das Fluidbereitstellsystem nach einem der vorhergehenden Ansprüche, wobei die Verkapselung (30') ferner aufweist:
eine Zugangsöffnung (200) für den Körper (90),
einen Verschluss (110), der in Bezug auf den Körper zwischen einer ersten und einer zweiten Verschlussposition bewegbar ist, wobei der Verschluss (110) die Zugangsöffnung blockiert, wenn sich dieser in der ersten Verschlussposition befindet, und wobei der Verschluss die Zugangsöffnung zu einem Inneren des Körpers freilegt, wenn sich dieser in der zweiten Verschlussposition befindet, und
eine erste Abschirmung, die der ersten Kammer (162) zugeordnet ist, wobei die erste Kammer mit der Zugangsöffnung ausgerichtet ist, wobei die Zugangsöffnung angepasst ist, um einen Kolben einer Einspritzvorrichtung aufzunehmen, um mit dem Plungerkolben der ersten Spritze (116) zusammenzuwirken.

14. Das Fluidbereitstellsystem nach Anspruch 13, das ferner ein Vorspannelement aufweist, das mit dem Verschluss (110) zusammenwirkt, um den Verschluss in die erste Verschlussposition vorzuspannen.

15. Das Fluidbereitstellsystem nach Anspruch 14, wobei der Verschluss eine radiologische Abschirmung aufweist.

16. Das Fluidbereitstellsystem nach Anspruch 13, das ferner eine Markierung (252) aufweist, die die Zugangsöffnung (220) der Verkapselung blockiert.

17. Das Fluidbereitstellsystem nach Anspruch 16, wobei die Markierung (252) eine radiologische Abschirmung aufweist.

18. Das Fluidbereitstellsystem nach Anspruch 17, wobei die Markierung (252) aus der Gruppe ausgewählt wurde, die aus einem Barcode, einem RFID-Tag oder einer vom Menschen lesbaren Markierung besteht.

19. Das Fluidbereitstellsystem nach einem der vorhergehenden Ansprüche, wobei der Körper (90) einen ersten und einen zweiten Körperabschnitt aufweist, die durch ein Gelenk (160) miteinander verbunden sind und in Bezug zueinander zwischen einer geöffneten und geschlossenen Position beweglich sind.

20. Das Fluidbereitstellsystem nach einem der vorhergehenden Ansprüche, wobei der Körper (90) eine dritte Kammer (263) aufweist, wobei das erste Fluidbereitstellsystm ferner eine zweite Spritze (262) aufweist, die sich in der dritten Kammer (263) befindet.

21. Das Fluidbereitstellsystem nach Anspruch 20, wobei die erste und die zweite Spritze in einen gemeinsamen Kanal der medizinischen Rohrleitung ausgeben.

## Revendications

1. Système d'administration de fluide (80'), comprenant :
une enceinte de confinement (30') comprenant un corps (90), dans lequel ledit corps comprend des première et deuxième chambres (162, 164) et un passage (190) qui s'étend entre lesdites première et deuxième chambres ;
une première seringue (116) disposée à l'intérieur de ladite première chambre (162) ; et
une tubulure médicale (118) comprenant des première et deuxième portions de conduit (118a, 118b), dans lequel ladite première portion de conduit (118a) est raccordée à ladite première seringue (116), dans lequel ladite tubulure médicale (118) s'étend par ledit passage (190), et dans lequel ladite deuxième portion de conduit (118b) est disposée à l'intérieur de ladite deuxième chambre (164) et est raccordée de manière à laisser passer un fluide à ladite première portion de conduit (118a),
ladite tubulure médicale (118) étant adaptée à une extraction hors de ladite enceinte de confinement (30') pour une opération d'administration de fluide tout en restant raccordée à ladite seringue (116).

2. Système d'administration de fluide selon la revendication 1, comprenant en outre un capuchon (104) raccordé de manière amovible audit corps de ladite enceinte de confinement.

3. Système d'administration de fluide selon la revendication 2, dans lequel une extrémité de ladite deuxième portion de conduit (118b) est couplée de manière amovible audit capuchon (104).

4. Système d'administration de fluide selon la revendication 3, dans lequel ledit capuchon (104) comprend une saillie (212) qui s'étend dans une extrémité ouverte de ladite deuxième portion de conduit (118b).

5. Système d'administration de fluide selon la revendication 3 ou la revendication 4, dans lequel ladite deuxième portion de conduit (118b) coopère avec une surface dudit capuchon (104) qui sert d'interface avec ladite deuxième chambre (164) lorsque ledit capuchon est monté sur ledit corps (90) de ladite enceinte de confinement (30').

6. Système d'administration de fluide selon l'une quelconque des revendications précédentes, comprenant en outre un blindage radiologique (130) pour ladite première chambre (162).

7. Système d'administration de fluide selon l'une quelconque des revendications précédentes, comprenant en outre une valve (177) disposée à l'intérieur de ladite tubulure médicale (118).

8. Système d'administration de fluide selon la revendication 7, dans lequel ladite tubulure médicale (118) contient un premier fluide, et dans lequel ladite première seringue (116) contient un deuxième fluide.

9. Système d'administration de fluide selon la revendication 8, dans lequel ledit premier fluide comprend une solution saline et dans lequel ledit deuxième fluide comprend un fluide radioactif.

10. Système d'administration de fluide selon l'une quelconque des revendications précédentes, dans lequel ledit corps (90) de ladite enceinte de confinement (30') comprend une portion d'extrémité à col (124) qui coïncide avec une extrémité de ladite deuxième chambre (164).

11. Système d'administration de fluide selon l'une quelconque des revendications précédentes, dans lequel ledit passage (190) comprend une première portion de passage qui s'étend depuis ladite première chambre (162) le long d'un axe longitudinal de ladite enceinte de confinement (30'), une deuxième portion de passage qui s'étend depuis ladite première portion de passage et s'éloigne dudit axe longitudinal, et une troisième portion de passage qui s'étend parallèlement à ladite première portion de passage, mais qui est décalée par rapport à celle-ci.

12. Système d'administration de fluide selon la revendication 11, dans lequel ladite enceinte de confinement (30') comprend en outre un deuxième blindage radiologique disposé autour dudit passage (190).

13. Système d'administration de fluide selon l'une quelconque des revendications précédentes, dans lequel ladite enceinte de confinement (30') comprend en outre :
une ouverture d'accès (220) pour ledit corps (90),
un obturateur (110) mobile par rapport audit corps (90) entre des première et deuxième positions d'obturateur, dans lequel ledit obturateur (110) bloque ladite ouverture d'accès quand il se trouve dans la première position d'obturateur, et dans lequel ledit obturateur expose ladite ouverture d'accès vers une partie intérieure dudit corps quand il se trouve dans la deuxième position d'obturateur ; et
un premier blindage associé à ladite première chambre (162), dans lequel ladite première chambre est alignée avec ladite ouverture d'accès, dans lequel ladite ouverture d'accès est adaptée pour recevoir un poussoir d'un dispositif d'injection destiné à interagir avec ledit piston de ladite première seringue (116).

14. Système d'administration de fluide selon la revendication 13, comprenant en outre un élément de sollicitation qui interagit avec ledit obturateur (110) pour solliciter ledit obturateur (110) dans ladite première position d'obturateur.

15. Système d'administration de fluide selon la revendication 14, dans lequel ledit obturateur comprend un blindage radiologique.

16. Système d'administration de fluide selon la revendication 13, comprenant en outre une étiquette (252) qui bloque ladite ouverture d'accès (220) de ladite enceinte de confinement.

17. Système d'administration de fluide selon la revendication 16, dans lequel ladite étiquette (252) comprend un blindage radiologique.

18. Système d'administration de fluide selon la revendication 17, dans lequel ladite étiquette (252) est sélectionnée dans le groupe composé d'un code à barres, d'une étiquette d'identification par radiofréquence, ou d'une étiquette lisible par l'utilisateur.

19. Système d'administration de fluide selon l'une quelconque des revendications précédentes, dans lequel ledit corps (90) comprend des première et deuxième portions de corps qui sont raccordées par une charnière (160), et mobiles l'une par rapport à l'autre entre des positions ouverte et fermée.

20. Système d'administration de fluide selon l'une quelconque des revendications précédentes, dans lequel ledit corps (90) comprend une troisième chambre (263), dans lequel ledit système d'administration de fluide comprend en outre une deuxième seringue (262) disposée à l'intérieur de ladite troisième chambre (263).

21. Système d'administration de fluide selon la revendication 20, dans lequel lesdites première et deuxième seringues se déchargent dans un conduit commun de ladite tubulure médicale.
